# EUROPEAN PATENT SPECIFICATION

(11) **EP 2 337 864 B1**
(45) Date of publication and mention of the grant of the patent: **26.11.2014**
(21) Application number: 09745014.2
(22) Date of filing: 26.10.2009
(51) Int. Cl.: C12Q 1/68, G01N 33/564

(54) **BIOMARKERS FOR PREDICTING THE DEVELOPMENT OF CHRONIC AUTOIMMUNE DISEASES**
BIOMARKER ZUR VORHERSAGE DER ENTWICKLUNG CHRONISCHER AUTOIMMUNKRANKHEITEN
BIOMARQUEURS POUR PRÉDIRE LE DÉVELOPPEMENT DE MALADIES AUTO-IMMUNES CHRONIQUES

(30) Priority: 24.10.2008 EP 08167570
(43) Date of publication of application: 29.06.2011
(73) Proprietor: Vereniging voor christelijk hoger onderwijs, Wetenschappelijk onderzoek en patiëntenzorg, 1081 HV Amsterdam (NL)
(72) Inventor: VERWEIJ, Cornelis, Lammert, 1391 CD Abcoude (NL); VAN SCHAARDENBURG, Dirkjan, 1215 BR Hilversum (NL); VAN BAARSEN, Elisabeth, Geertruida, Maria, 1135 AN EDAM (NL)
(74) Representative: Jansen, Cornelis Marinus
(86) International application number: PCT/EP2009/064099
(87) International publication number: WO 2010/046503

(56) References cited:
- WO-A2-02/48310
- SARAUX A ET AL: "Value of laboratory tests in early prediction of rheumatoid arthritis" ARTHRITIS AND RHEUMATISM, LIPPINCOTT, PHILADELPHIA, US, vol. 47, no. 2, 15 April 2002 (2002-04-15), pages 155-165, XP002407373 ISSN: 0004-3591
- VAN BAARSEN LISA G M ET AL: "Altered innate immune response in a subgroup of individuals at risk for rheumatoid arthritis" ARTHRITIS AND RHEUMATISM, LIPPINCOTT, PHILADELPHIA, US, vol. 58, no. 9, SUPPL, 1 September 2008 (2008-09-01), page S616, XP008105285 ISSN: 0004-3591 [retrieved on 2008-08-29]
- RANTAPÄÄ-DAHLQVIST SOLBRITT ET AL: "Antibodies against cyclic citrullinated peptide and IgA rheumatoid factor predict the development of rheumatoid arthritis" ARTHRITIS AND RHEUMATISM, LIPPINCOTT, PHILADELPHIA, US, vol. 48, no. 10, 1 October 2003 (2003-10-01), pages 2741-2749, XP002333657 ISSN: 0004-3591 cited in the application
- VAN DER POUW KRAAN T C T ET AL: "Rheumatoid arthritis subtypes identified by genomic profiling of peripheral blood cells: assignment of a type I interferon signature in a subpopulation of patients" ANNALS OF THE RHEUMATIC DISEASES, BRITISH MEDICAL ASSOCIATION, LONDON, GB, vol. 66, no. 8, 1 August 2007 (2007-08-01), pages 1008-1014, XP008103935 ISSN: 0003-4967 [retrieved on 2007-01-18] cited in the application
- FENG XUEBING ET AL: "Association of increased interferon-inducible gene expression with disease activity and lupus nephritis in patients with systemic lupus erythematosus." ARTHRITIS AND RHEUMATISM SEP 2006, vol. 54, no. 9, September 2006 (2006-09), pages 2951-2962, XP002561971 ISSN: 0004-3591
- OLOFSSON PETER ET AL: "Arthritis suppression by NADPH activation operates through an interferon-[beta] pathway" BMC BIOLOGY, BIOMED CENTRAL, LONDON, GB, GB, vol. 5, no. 1, 9 May 2007 (2007-05-09), page 19, XP021024979 ISSN: 1741-7007
- J B Andersen ET AL: "Stage-associated overexpression of the ubiquitin-like protein, ISG15, in bladder cancer", British Journal of Cancer, vol. 94, no. 10, 25 April 2006 (2006-04-25), pages 1465-1471, XP055113960, ISSN: 0007-0920, DOI: 10.1038/sj.bjc.6603099

## Description

### Field of the invention

The invention relates to methods for determining whether an individual has an increased risk of developing chronic autoimmune diseases.

### Background of the invention

Autoimmune diseases are generally understood to be diseases where the target of the disease is "self" or "self antigen". Among the many types of autoimmune diseases, there are a number of diseases that are believed to involve T cell immunity directed to self antigens, including, for example, multiple sclerosis (MS), Type I diabetes, and rheumatoid arthritis (RA).

RA is a chronic inflammatory disorder characterized by joint pain. The course of the disease is variable, but can be both debilitating and mutilating. According to conservative estimates approximately 50,000,000 individuals are afflicted with RA worldwide. Those individuals are not only subjected to life-long disability and misery, but as current evidence suggests, their life expectancy is compromised as well. Systemic lupus erythematosus (SLE) is a chronic inflammatory disease that can affect various parts of the body including skin, blood, kidneys, and joints. SLE may manifest as a mild disease or be serious and life-threatening. More than 16,000 cases of SLE are reported in the United States each year, with up to 1.5 million cases diagnosed.

Although SLE can occur at any age, and in either sex, it has been found to occur 10-15 times more frequently in women.

SLE is characterized by the production of auto-antibodies having specificity for a wide range of self-antigens. SLE auto-antibodies mediate organ damage by directly binding to host tissues and by forming immune complexes that deposit in vascular tissues and activate various immune cells. SLE induced damage to the host targets the skin, kidneys, vasculature, joints, various blood elements, and the central nervous system (CNS). The severity of disease, the spectrum of clinical involvement, and the response to therapy vary widely among patients. The clinical heterogeneity of SLE makes it challenging to diagnose, monitor and manage.

When a patient is diagnosed with an autoimmune disease such as RA and SLE, the choice of appropriate therapeutic interventions would be considerably facilitated by diagnostic and prognostic indicators that accurately predict future severity. WO 02/48310 A2 (Genetics Institute LLC, 20.6.2002) discloses diagnostic assays and markers of rheumatoid arthritis.

Thus, there is a need in the art for reliable prognostic methods to predict suffering from chronic autoimmune diseases in individuals.

### Summary of the invention

The present inventors have determined that a number of genes are indicative of an increased risk of developing chronic autoimmune diseases, in particular Rheumatoi Arthritis (RA). They found that the occurrence of RA may be predicted even long before an individual presents with symptoms of RA. A set of genes that may be used in the invention is presented herein, in particular a gene selected from the group consisting of genes ISG15, EPSTI1, IFI6, OAS3, IFI44L, RSAD2, IFIT1, MX1, CD274, SERPING1, IFI27, CD19, CD79A, CD79B, MS4A1, FCRL5, DARC, BCL2L1, RBM38, BAG1, TESC, KLF1, ERAF, SELENBP1, CCL5, IFNG, GZMH and NKG7 was found to be useful in a method according to the invention.

Hence, the invention relates to an in vitro method for determining whether an individual has an increased risk of developing symptoms of Rheumatoid Arthritis comprising the steps of determining the expression level of at least the ISG15 gene in a sample obtained from said individual and comparing said expression level with a predetermined reference value wherein an increased level of expression of said ISG15 gene in said sample is indicative of an increased risk.

### Detailed description of the invention

In a broad sense, a method is provided for determining whether an individual has an increased risk of developing symptoms of a chronic autoimmune disease within a time frame of 10 years after the completion of said method compared to the average risk of a reference population, comprising determining in a sample of said individual the levels of expression products of a collection of genes and determining whether the expression products are present at a level that is different when compared to the level of the same expression products of a control.

Upon analysis of the gene set indicative of an increased risk as shown in table 8, it was found that the genes clustered into 4 distinct categories. They appeared to be involved in (i) IFN-mediated immunity, (ii) hematopoiesis, (iii) B-cell mediated immunity and/or (iv) cytokine mediated immunity.

It is therefore postulated that other genes involved in those cellular processes which are not necessarily shown in table 8 will also prove to be indicative of an increased risk of developing chronic autoimmune diseases, in particular RA.

Higher levels of expression products of genes involved in IFN-mediated immunity, hematopoiesis and cytokines are predictive for an increased risk and higher levels of expression products of genes involved in the cellular process of B-cell mediated immunity are predictive for a decreased risk.

A method according to the invention may be used to determine an increased risk of developing symptoms of a chronic autoimmune disease within a time frame of 10 years after the completion of said method.

The expression "a predetermined reference value" in this context means that the expression level of a particular gene is determined in at least one sample from a normal individual, more in particular an individual that has proven not to develop any symptoms of RA in a number of years after the sample was taken from the individual, in particular wherein the number of years is more than 5 years, such as more than 10 years. Preferably, the reference value is an average of more than one such as 2, 3, 4,or more than 5 samples taken from different normal individuals.

Preferred samples include whole blood, saliva, faecal material, buccal smears, skin, and biopsies of specific organ tissues, such as muscle or nerve tissue and hair follicle, because these samples comprise relevant expression products. Preferably, said sample comprises a cell sample, because cell samples comprise proteins and nucleic acids. Most preferably, the biological sample is a blood sample, because a blood sample is easy obtainable and comprises large amounts of relevant expression products.

Expression products comprise nucleic acids and proteins. Nucleic acids in the invention preferably comprise RNA. The levels of the expression products may be determined separately for each different expression product or as a single measurement for more different expression products simultaneously. Preferably, the determination of the level of the expression products is performed for each different expression product separately, resulting in a separate measurement of the level of the expression product for each different expression product. This enables a more accurate comparison of expression levels of expression products with the expression levels of the same expression products in a control. A control may be a single individual or group comprising of at least two individuals, preferably four individuals. If the expression level is determined of an expression product from more than one individual, usually the median or mean expression level of these individuals is used for comparison.

Determination of the level of the expression products according to a methods of the invention may comprise the measurement of the amount of nucleic acids or of proteins. In a preferred embodiment of the invention, determination of the level of the expression products comprises determination of the amount of RNA, preferably mRNA. A level can be the absolute level or a relative level compared to the level of another mRNA. mRNA can be isolated from the samples by methods well known to those skilled in the art as described, e.g., in Ausubel et al., Current Protocols in Molecular Biology, Vol. 1 , pp.4.1.1 -4.2.9 and 4.5.1-4.5.3, John Wiley & Sons, Inc. (1996). Methods for detecting the amount of mRNA are well known in the art and include, but are not limited to, northern blotting, reverse transcription PCR, real time quantitative PCR and other hybridization methods. The amount of mRNA is preferably determined by contacting the mRNAs with at least one sequence-specific oligonucleotide which hybridises to said mRNA. In a preferred embodiment said mRNA is determined with two sequence-specific oligonucleotides which hybridise to different sections of said mRNA. The sequence-specific oligonucleotides are preferably of sufficient length to specifically hybridize only to the RNA or to a cDNA prepared from said mRNA. As used herein, the term "oligonucleotide" refers to a single-stranded nucleic acid. Generally the sequence-specific oligonucleotides will be at least 15 to 20 nucleotides in length, although in some cases longer probes of at least 20 to 25 nucleotides will be desirable. Said sequence-specific oligonucleotides may also comprise non-specific nucleic acids. Such non-specific nucleic acids can be used for structural purposes, for example as an anchor to immobilise the oligonucleotides. The sequence-specific oligonucleotide can be labelled with one or more labelling moieties to permit detection of the hybridized probe/target polynucleotide complexes. Labelling moieties can include compositions that can be detected by spectroscopic, biochemical, photochemical, bioelectronic, immunochemical, and electrical optical or chemical means. Examples of labelling moieties include, but are not limited to, radioisotopes, e.g., 32P, 33P, 35S, chemiluminescent compounds, labelled binding proteins, heavy metal atoms, spectroscopic markers such as fluorescent markers and dyes, linked enzymes, mass spectrometry tags, and magnetic labels. Oligonucleotide arrays for mRNA or expression monitoring can be prepared and used according to techniques which are well known to those skilled in the art as described, e.g., in Lockhart et al., Nature Biotechnology, Vol. 14, pp. 1675-1680 (1996); McGall et al., Proc. Natl. Acad. Scl. USA, Vol. 93, pp. 13555-13460 (1996); and U.S. Patent No. 6,040,138.

A preferred method for determining the amount of mRNA involves hybridization of labelled mRNA to an ordered array of sequence-specific oligonucleotides. Such a method allows the simultaneously determination of the mRNA amounts. The sequence-specific oligonucleotides utilized in this hybridization method typically are bound to a solid support. Examples of solid supports include, but are not limited to, membranes, filters, slides, paper, nylon, wafers, fibers, magnetic or nonmagnetic beads, gels, tubing, polymers, polyvinyl chloride dishes, etc.

According to a preferred embodiment of the invention the determining the level(s) of the expression products is performed by measuring the amount of protein. The term "protein" as used herein may be used synonymously with the term "polypeptide" or may refer to, in addition, a complex of two or more polypeptides which may be linked by bonds other than peptide bonds, for example, such polypeptides making up the protein may be linked by disulfide bonds. The term "protein" may also comprehend a family of polypeptides having identical amino acid sequences but different post-translational modifications, particularly as may be added when such proteins are expressed in eukaryotic hosts. These proteins can be either in their native form or they may be immunologically detectable fragments of the proteins resulting, for example, from proteolytic breakdown. By "immunologically detectable" is meant that the protein fragments comprise an epitope which is specifically recognized by e.g. mass spectrometry or antibody reagents as described below. Proteins levels can be determined by methods known to the skilled person, comprising but not limited to: mass spectrometry, Western blotting, immunoassays, protein expression assay, protein microarray etc.

A preferred embodiment of the invention provides a protein microarray (Templin et al. 2004; Comb.Chem.High Throughput Screen., vol. 7, no. 3, pp. 223-229) for simultaneous binding and quantification of the at least two biomarker proteins according to the invention. The protein microarray consists of molecules (capture agents) bound to a defined spot position on a support material. The array is then exposed to a complex protein sample. Capture agents such as antibodies are able to bind the protein of interest from the biological sample. The binding of the specific analyte proteins to the individual spots can then be monitored by quantifying the signal generated by each spot (MacBeath 2002; Nat. Genet, vol. 32 Suppl, pp. 526-532; Zhu & Snyder 2003; Curr.Opin.Chem.Biol., vol. 7, no. 1 , pp. 55-63). Protein microarrays can be classified into two major categories according to their applications. These are defined as protein expression microarrays, and protein function microarrays (Kodadek 2001 ; Chem.Biol., vol. 8, no. 2, pp. 105-115). Protein expression microarrays mainly serve as an analytic tool, and can be used to detect and quantify proteins, antigen or antibodies in a biological fluid or sample. Protein function microarrays on the other hand can be used to study protein-protein, enzyme-substrate and small molecule-protein interactions (Huang 2003; Front Biosci., vol. 8, p. d559-d576). Protein microarrays also come in many structural forms. These include two-dimensional microarrays constructed on a planar surface, and three-dimensional microarrays which use a Flow- through support.

Types of protein microarray set-ups: reverse phase arrays (RPAs) and forward phase arrays (FPAs) (Liotta et al. 2003; Cancer Cell, vol. 3, no. 4, pp. 317-325). In RPAs a small amount of a tissue or cell sample is immobilized on each array spot, such that an array is composed of different patient samples or cellular lysates. In the RPA format, each array is incubated with one detection protein (e.g., antibody), and a single analyte endpoint is measured and directly compared across multiple samples. In FPAs capture agents, usually an antibody or antigen, are immobilized onto the surface and act as a capture molecule. Each spot contains one type of immobilized antibody or capture protein. Each array is incubated with one test sample, and multiple analytes are measured at once.

One of the most common forms of FPAs is an antibody microarray. Antibody microarrays can be produced in two forms, either by a sandwich assay or by direct labelling approach. The sandwich assay approach utilizes two different antibodies that recognize two different epitopes on the target protein. One antibody is immobilized on a solid support and captures its target molecule from the biological sample. Using the appropriate detection system, the labelled second antibody detects the bound targets. The main advantage of the sandwich assay is its high specificity and sensitivity (Templin, Stoll, Bachmann, & Joos 2004; Comb.Chem.High Throughput.Screen., vol. 7, no. 3, pp. 223-229). High sensitivity is achieved by a dramatic reduction of background yielding a high signal-to noise ratio. In addition, only minimal amounts of labelled detection antibodies are applied in contrast to the direct labelling approach were a huge amount of labelled proteins are present in a sample. The sandwich immunoassay format can also be easily amenable to the field of microarray technology, and such immunoassays can be applied to the protein microarray format to quantify proteins in conditioned media and/or patient sera (Huang et al. 2001 ; Clin.Chem.Lab Med., vol. 39, no. 3, pp. 209-214; Schweitzer et al. 2002; Nat Biotechnol., vol. 20, no. 4, pp. 359-365).

In the direct labelling approach, all proteins in a sample are labelled with a fluorophore. Labelled proteins that bind to the protein microarray such as to an antibody microarray are then directly detected by fluorescence. An adaptation of the direct labeling approach is described by Haab and co-workers (Haab, Dunham, & Brown 2001; Genome Biol., vol. 2, no. 2, p). In this approach, proteins from two different biological samples are labelled with either Cy3 or Cy5 fluorophores. These two labelled samples are then equally mixed together and applied to an antibody microarray. This approach, for example, allows comparisons to be made between diseased and healthy, or treated and untreated samples. Direct labelling has several advantages, one of which is that the direct labelling method only requires one specific antibody to perform an assay.

Miniaturized and multiplexed immunoassays may also used to screen a biological sample for the presence or absence of proteins such as antibodies (Joos et al. 2000; Electrophoresis, vol. 21 , no. 13, pp. 2641-2650; Robinson et al. 2002; Nat.Med., vol. 8, no. 3, pp. 295-301).

In a preferred embodiment of the invention, the detection or capture agents such as the antibodies are immobilized on a solid support, such as for example on a polystyrene surface. In another most preferred embodiment, the detection or capture agents are spotted or immobilized in duplicate, triplicate or quadruplicate onto the bottom of one well of a 96 well plate.

An increased expression level of a particular gene or group of genes may also be determined by measuring other factors than RNA or protein directly expressed by the gene. WO 2008/147206 provides a number of polymorphisms that can be used to determine an increased interferon signature. In particular, the invention relates to a method for classifying an individual as preclinical RA patient with an increased type 1 interferon response signature, said method comprising the steps of determining in a nucleic acid sample from said individual one or more polymorphisms in the IFR5 gene related to an increased type 1 interferon gene signature and determining, based on the genotype of said polymorphism(s), if the individual has increased type 1 interferon response signature.

In a preferred embodiment said one or more polymorphisms comprises a polymorphism in SNP rs2004640, in SNP rs4728142, or in a 30 bp insertion-deletion polymorphism in exon 6.

We found that the T -allele and in particular the TT genotype of rs2004640 and/or the A-allele and in particular the AA genotype of rs4728142 is associated with an increased IFN response signature in the blood. Also patients homozygous for the 5bp CGGGG deletion show a increased IFN response activity response.

In a preferred embodiment more than one polymorphism is determined to indicate whether an individual is a preclinical RA patient. It is preferred to determine the haplotype for the indicated polymorphisms and classify the cells of the individual on the basis of of the determined haplotype. In this embodiment Haplotype A (rs4728142 (A) rs2004640 (T) exon 6 indel (del) rs10954213 (A)) associated with an increased risk and Haplotype B (rs4728142 (G) rs2004640 (G) exon 6 indel (in) rs10954213 (G)) is associated with prevention to develop arthritis.

Thus in one aspect the invention provides a method wherein a polymorphism is used to indicate whether an individual is likely to be a preclinical RA patient. In a preferred embodiment said polymorphism is a polymorphism of rs4728142, rs2004640, exon 6 indel (del) or exon 6 indel (in), or rs10954213. In a preferred embodiment the haplotype for at least two and preferably at least 3 and more preferably all of the polymorphisms mentioned are determined.

The polymorphism may also determined in a method according to the invention by analysis of the complementary strand. In this case, the correlations and predictions as indicated herein above, are of course associated with the presence of the respective complementary nucleotide(s).

The combining feature is that these polymorphisms are all situated in or close by the IRF5 gene.

The invention further provides the use of a polymorphism that discriminates alleles of the IFR5 gene and that correlates for more than 90% with a polymorphism associated with development of RA (preclinical RA patient), for classifying an individual as preclinical RA patient.

In a method according to the invention, an individual has an increased risk of developing the symptoms of a chronic autoimmune disease if expression level of the expression products of a said collection of genes are different compared to the levels of the same expression products of a control. An expression level is classified as different when said expression level of said expression product is statistically significantly increased or decreased in said individual compared to the level of the same expression product found in control individuals. The term "significantly" or "statistically significant" refers to statistical significance and generally means a two standard deviation (SD) above normal, or higher, or below, or lower concentration of the expression product. In preferred embodiments, said difference is classified as statistically significant if the expression level is at least a 20 percent increased or decreased compared to expression level of the same expression product in control individuals. Preferably, the increase or decrease is at least 20, 25, 30, 35, 40, 45, 50, 75, 100, 150, 200 or 250 percent. Most preferably, said increase or decrease is at least 100 percent.

It is also possible to determine an increased risk of developing the symptoms of a chronic autoimmune disease by determining if the number of expression products having a different expression level, differs significantly from the same number in control individuals. Preferably, said numbers are compared within the same collection of genes involved in the same cellular process. Said numbers can be related to the total number of expression products of said collection of genes from which the levels of expression products are determined or a selection thereof. It is preferred that said numbers are compared to numbers of control individuals. Said levels can be compared with cut-off levels of reference intervals of said expression products, wherein said reference intervals are based on the levels of the same expression products in a control group. Said cut-off levels are usually the values corresponding to 5% or 95% of a reference interval (which is usually a statistically determined confidence interval) of the levels the expression products in a control group. Alternatively, a cut-off level can also be based on the level of the expression products in a control individual or a control group. Preferably the cut-off level is at least a factor two higher or lower than the level of the same expression product in a control individual or mean or median level of the expression product in a control group. The number of said levels is determined that are outside said intervals within said at least one collection of genes. Said number is compared with a cut off value, wherein said cut off value is based on the number of levels of the same expression products that are outside said intervals in a control group within said at least one collection of genes, wherein a higher number than said cut off value is predictive for an increased risk.

Preferably said at least one gene comprises at least 10 genes, more preferably at least 15, 20, 25, 30, 35, 45 or 52 genes of Table 8. If more genes are used in the method, the method will generate less false positive and false negative results.

In a preferred embodiment, the chronic autoimmune disease is Rheumatoid Arthritis (RA) or Systemic Lupus Erythematosus (SLE). The method is very predictive for these diseases. Preferably, said autoimmune disease is RA.

Even more preferred is a method, wherein said genes involved in the cellular process of IFN-mediated immunity comprise at least 1 gene from
Table 4. More preferably 2, 3, 5, 7, 10, 16 genes of
Table 4. If more genes involved in IFN-mediated immunity are used, the method is more accurate.

Another more preferred embodiment is a method, wherein said genes involved in the cellular process of B-cell mediated immunity comprise at least 1 gene from Table 5. More preferably 2, 3, 5, 7, 10 genes of Table 5. If more genes involved in B-cell mediated immunity are used, the method is more accurate.

Another more preferred embodiment is a method, wherein said genes involved in the cellular process of hematopoiesis comprise at least 1 gene from Table 6. More preferably 2, 3, 5, 7, 10 genes of Table 6. If more genes involved in hematopoiesis are used, the method is more accurate.

Another more preferred embodiment is a method, wherein said genes involved in the cellular process of cytokine mediated immunity comprise at least 1 gene from Table 7, more preferably 2, 3, 5, 7, 9 genes of Table 7. If more genes involved in cytokine mediated immunity are used, the method is more accurate.

Another preferred embodiment is a method, wherein said genes are selected from the group comprising ISG15, EPSTI1, IFI6, OAS3, IFI44L, RSAD2, IFIT1, MX1, CD274, SERPING1, IFI27, CD19, CD79A, CD79B, MS4A1, FCRL5, DARC, BCL2L1, RBM38, BAG1, TESC, KLF1, ERAF, SELENBP1, CCL5, IFNG, GZMH and NKG7. When using these genes, the method is more accurate.

Another preferred embodiment is a method wherein said collection of genes comprises genes coding for a secreted protein. An advantage thereof is that this method is very well suited for determining the risk based on a secreted protein as indicated in Table 8. Said secreted protein can easily be obtained. The levels of said secreted protein can be compared with control levels. Significantly different levels of a secreted protein are predictive of an increased risk of developing RA.

Another preferred embodiment is a method wherein said collection of genes comprises genes coding for a membrane bound protein. An advantage thereof is that this method is very well suited for determining risk using for example FACS analysis. Levels of membrane bound proteins can be established using a FACS and compared to a control and determine whether said level is elevated. It is also possible to determine said levels in cellular subsets based on the expression of surface markers.

Also preferred is a method as described above, further comprising testing a sample of said individual for a further factor associated with an increased risk for developing RA. By combining the method with testing a further factor that is associated with an increased risk for developing RA, the method is more accurate. Any said further factor can be used. Preferably, said further factor comprises detecting the presence of an anti-citrullinated protein/peptide antibody, and/or rheumatoid factor (RF). An advantage is that risk of developing RA within 2-5 years when using a combined test using ACPA and/or RF with the method according to the invention is increased to 95-100%. Examples of anti-citrullinated protein/peptide antibody are antiperinuclear factor (APF, Nienhuis and Mandema, Ann. Rheum Dis 1964; 23: 302-5), antikeratin antibody (AKA, Aho KJ. Rheumatol 1993; 20:1278-8). These antibodies bind to substrates containing modified cittruline (Schellekens et al. J. Clin Invest 1998; 101: 273-281. Methods of using anti-citrullinated protein/peptide antibody to predict RA are described in Meyer et al., Ann Rheum Dis 2003;62:120-126 or Alexiou et al. Clinical rheumatology 2008, vol. 27, no4, pp. 511-513 or Alexiou et al. Clinical rheumatology 2008, vol. 27, no4, pp. 511-513. A method to use RF for the prediction of RA is described in Rantapaa-Dahlqvist 2003 Arthritis Rheum 48: 2741-2749 and Nielen et al. Arthritis Rheum. 2004 Aug;50(8):2423-7. Such further factor may also comprise detecting an elevated level of MCP-1 , IL-10, FGF2 and/or Flt-3L or a polymorphism of a gene associated with susceptibility to RA. MCP-1 polymorphism is associated with the susceptibility to RA in patients lacking the HLA SE. Therefore, if an individuals is further tested on the presence of an MCP-1 polymorphism (described in González-Escribano et al., Human Immunology Volume 64, Issue 7, July 2003, Pages 741-744), the method of the invention will be more powerful. The use of for the presence of polymorphisms of the IL-10 gene for determining the risk of developing RA in an individual is described in MacKay Rheumatology 2003; 42: 149-153. In addition, synovial fluid (SF) levels of IL-10 are increased in patients of early RA (G A Mittal and VR Joshi J Indian Rheumatol Assoc 2002: 10 : 59 - 60). The levels of IL-10 in SF can also be determined as a further factor in the method. FLT3-mediated conditions in autoimmune diseases are described in WO/2008/016665 and WO/2002/067760.

Preferably, a method according to the invention is combined with a medical treatment of teh patient. Preferably, the patient is treated without any clinical symptoms being apparent. Such may be done by administering to said individual a composition for the treatment or prevention of RA. Said composition may be any composition that is used for the treatment or prevention of RA. A non limiting list of treatments comprises:
a) nonsteroidal anti-inflammatory drugs (NSAIDs);
b) disease-modifying anti-rheumatic drugs (DMARDs);
c) steroids; and
d) analgesics.

Preferably, said administration is done at a time point when said individual does not suffer from a clinical symptom of RA. An advantage thereof is that treatment of RA is more successful if it is started before symptoms have developed.

### Figure legends

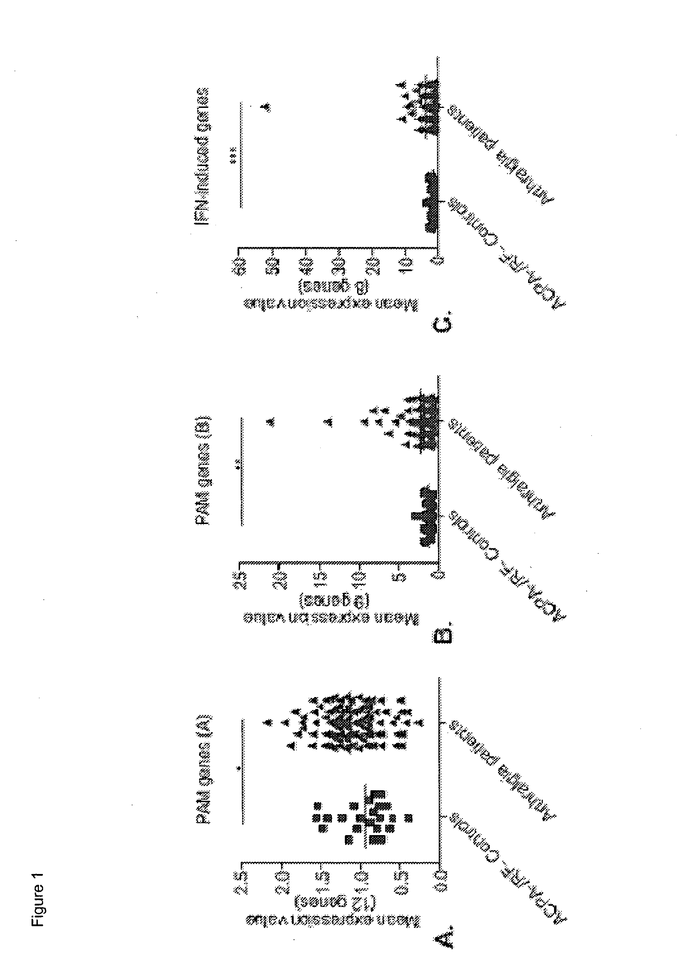

Figure 1. Confirmation of microarray data in a larger group of autoantibody positive arthralgia patients
   Gene expression levels for the selected genes in Table 2 were measured in the total cohort of autoantibody positive arthralgia patients (n=109) and compared to ACPA and RF negative healthy controls (n=25). For each sample the mean expression values were calculated for the genes selected from the two different PAM analyses i.e. 6 controls vs. 19 autoantibody positive arthralgia patients (A) and 6 controls vs. 6 ACPA+RF- arthralgia patients (B) as well as for the IFN-induced gene set (C). Mean expression values of each gene set were compared between autoantibody positive arthralgia patients and healthy controls.
   * P < 0.05; ** P < 0.01; *** P < 0.001; ns not significant.

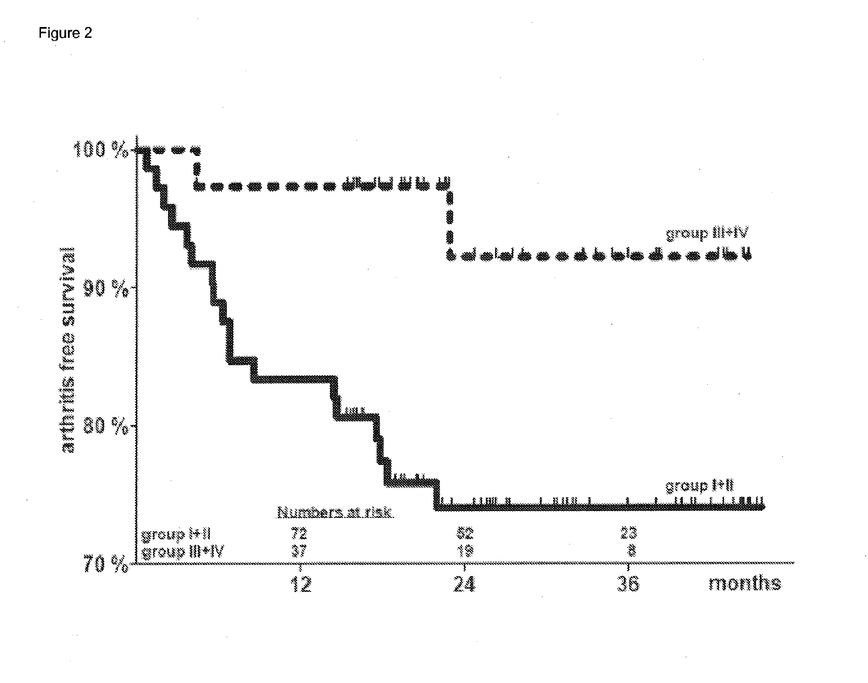

Figure 2. Kaplan Meier survival curve
   Arthritis free survival in ACPA and/or IgM-RF positive arthralgia patients, stratified for gene expression profiles I+II versus III+IV, as described in the examples section.
Figure 3. Kaplan Meier survival curve
   Arthritis free survival in ACPA and/or IgM-RF positive arthralgia patients, stratified for gene expression profiles I+II+IV versus III, as described in the examples section

### Examples

### Example 1 Study population

Between September 2004 and March 2007, ACPA and/or IgM-RF positive arthralgia patients were included for prospective follow-up of arthritis development. Inclusion and exclusion criteria for this cohort have been described previously [19]. In summary, a trained medical doctor (WB) and a senior rheumatologist (DS) independently scored for absence of arthritis (swollen joint count [SJC] =0) in 44 joints at physical examination at the baseline visit [20]. The senior rheumatologist was blinded for the reported joint complaints and the autoantibody status. Exclusion criteria were: arthritis revealed by chart review or baseline physical examination, erosions on hand or feet X-ray examination and previous treatment with a disease modifying anti-rheumatic drug (DMARD). In total, 109 patients were available for analysis. Arthritis development during follow-up was defined as a SJC of ≥ 1 and was independently confirmed by both physicians.

For comparison, 25 ACPA and IgM-RF negative healthy lab donors and 25 RA patients with established disease were included. The RA patients consisted of 25 randomly selected patients starting anti-TNF treatment at the Jan van Breemen Institute. All fulfilled the ACR criteria for RA [21] and the median disease duration was 11 years. As expected, only the age of the RA patients was different from controls and autoantibody positive arthralgia patients.

An overview of the subjects' characteristics is given in Table 1.

### Example 2 Serological measurements

Baseline laboratory parameters (determined batch wise at the end of the study period using the blood samples obtained at inclusion) included IgM-RF by in house enzyme-linked immunosorbent assay (ELISA) and ACPA by ELISA (second generation anti-CCP ELISA, Axis Shield, Dundee, United Kingdom). IgM-RF was calibrated with a national reference serum containing 200 IU/ml [22]. The cut-off level for IgM-RF antibody positivity was set at 30 IU/ml determined on the basis of ROC curves as described previously [6]. The cut-off level for ACPA positivity was set at 5 Arbitrary Units/ml (AU/ml) according to the manufacturer's instructions. High sensitive CRP levels were measured using CRPHS reagents with module C501 on a COBAS 6000 platform (Roche Diagnostics GmbH, Mannheim, Germany).

### Example 3 Blood sampling for RNA isolation

2.5 ml blood was drawn at baseline in PAXgene blood RNA isolation tubes (PreAnalytix, GmbH, Germany) and stored at -20°C. Tubes were thawed for 2 hours at room temperature prior to RNA isolation. Next, total RNA was isolated using the PAXgene RNA isolation kit according to the manufacturer's instructions including a DNAse (Qiagen, Venlo, Netherlands) step to remove genomic DNA.

### Example 4 Sample hybridisation for microarray analysis

We used 43K cDNA microarrays from the Stanford Functional Genomics Facility (http://microarray.org/sfgf/) printed on aminosilane-coated slides containing -20.000 unique genes. Only one batch of arrays was used for all experiments. First DNA spots were UV-crosslinked to the slide using 150-300 mJoules. Prior to sample hybridization, slides were pre-hybridized at 42 degrees Celsius for 15 minutes in a solution containing 40% ultra-pure formamide (Invitrogen, Breda, Netherlands), 5% SSC (Biochemika, Sigma), 0.1% SDS (Fluka Chemie, GmbH, Switzerland) and 50 g/ml BSA (Panvera, Madison, USA). After pre-hybridization slides were briefly rinsed in MilliQ water, thoroughly washed in boiling water and 95% ethanol and air-dried. Sample preparation and microarray hybridization was performed as described previously [23] apart from the different post-processing and pre-hybridization described above.

### Example 5 Microarray data analysis

Data storage and filtering was performed using the Stanford Microarray Database (SMD at: http://genome-www5.stanford.edu//) [24] as described previously [25]. Raw data can be downloaded from the publicly accessible Stanford database website. We used the Q-score tool from the database as a quality measure to remove low quality spots. Q-score determined the appropriate filter criteria for: the regression correlation between channels 1 and 2, the background settings and the minimal channel intensities. After removing the low quality spots, data values with the same Unigene Identifier were averaged and all array data was median centered (genes and arrays) resulting in good quality data for 19,648 gene transcripts. Statistical Analysis of Microarrays (SAM) [26] was used to determine significantly differential expressed genes. A gene was considered as significantly differential expressed if the False Discovery Rate (FDR) was equal to or less than 5%. Cluster analysis [27] was used to define clusters of coordinately expressed genes after which the data was visualized using Treeview. PANTHER (Protein ANalysis THrough Evolutionary Relationships) Classification System (Applied Biosystems, Foster City, CA, USA) was used at http://PANTHER.appliedbiosystems.com [28,29] to interpret our data. This analysis uses the binomial statistics tool to compare the list of significantly up- or downregulated genes to a reference list in order to statistically determine over- or under representation of PANTHER classification categories such as biological processes. A Bonferroni correction was applied to correct for multiple testing and a significant p-value (p<0.05) indicates that a given category may be of biological interest. For comparison the Ontology TermFinder tool from the SMD database was used, which, in the same manner as PANTHER when given a list of genes, identifies significantly over- or under represented gene ontology terms (using an FDR cut-off value of 5%). Both the PANTHER and TermFinder tools use Gene Ontology terms ([30] at http://www.geneontology.org/), but the PANTHER classification is greatly abbreviated and simplified to facilitate high-throughput analyses.

### Example 6 Selecting genes for validation

Microarray analysis revealed 6313 significantly differential expressed genes between autoantibody negative healthy individuals and arthralgia patients positive for ACPA and/or RF (supplementary Table S1). In order to narrow down the list of genes for validation, class prediction analysis of microarrays (PAM) was applied using the gene expression data as a training set. Applying a ten-fold cross validation this analysis identified a set of only 17 genes that could correctly classify our controls from autoantibody positive arthralgia patients while only two of the 19 arthralgia patients were classified as controls. Thus, the expression of these 17 genes could predict with a class error rate of only 10.5% if a sample was derived from an autoantibody negative control or an autoantibody positive arthralgia patient at risk of developing RA. Although this analysis could predict the sample class rather well, the proportions between the two sample groups are very unbalanced. Therefore we performed a second PAM analysis in which only data was used of the 6 autoantibody negative controls and the 6 ACPA+ but RF negative persons at risk resulting in a more balanced analysis. Strikingly, the expression of 14 genes could correctly classify these two different groups with a class error rate of 0%. Interestingly, the majority of these 14 genes are interferon-induced and only one gene overlapped with the first PAM analysis. When we performed a PAM analysis between the 6 controls and the 9 ACPA-RF+ patients, most of the genes overlapped with the first PAM analysis (6 controls versus 19 autoantibody positive arthralgia patients). Genes from both PAM analyses were selected for confirmation analysis in a larger cohort using the Taqman Low Density Array (TLDA) technology which is based on quantitative real-time PCR. Available predesigned Taqman primers and probes were used to validate gene expression levels of the above PAM analyses. For 10 selected genes no predesigned TLDA assays were available and therefore these genes were excluded from analysis.

We included 20 IFN related genes derived from comparisons between active RA patients and healthy controls performed previously [23]. In addition, IFN specific genes IFNA2, IFNb1 and IFNg were included and housekeeping genes GAPDH and 18SRNA were added for normalization. Detailed information for selected target genes is listed in Table 2.

### Example 7 Taqman® Low Density Arrays (TLDA)

Per TLDA card it is possible to analyze the expression of 48 genes in eight samples simultaneously. The expression of selected target genes (Tables 2 and 3) was validated in the total study cohort using TLDA (Applied Biosystems). Corresponding predesigned primers and probes (Table S2) were selected from the Applied Biosystems database to set up custom TLDA cards. In 160 samples randomly dispersed over the TLDA cards the expression levels of selected genes were measured at the outsourcing company ServiceXS B.V. (Leiden, Netherlands). Total RNA (0.5 µg) was reverse transcribed into cDNA using a Revertaid H-minus cDNA synthesis kit (MBI Fermentas, St. Leon-Rot, Germany) according to the manufacturer's instructions. From each sample diluted cDNA corresponding to 100 ng total RNA was used per TLDA card. For normalization the housekeeping genes GAPDH and 18SRNA were included in the analysis. Since the inter-sample variation in expression for GAPDH was much higher than for 18SRNA, the latter was chosen for normalization. In addition, the correlation between array and TLDA data was much better with 18SRNA normalized data (data not shown). An arbitrarily chosen ACPA-/RF- control sample was selected as calibrator sample. Data was analyzed using RQ manager 1.2 (Applied Biosystems) and since this program can only analyze 10 TLDA cards in one experiment, the calibrator sample was analyzed in duplicate on two different TLDA cards (Pearson R=0.9936 between the two experiments).

### Example 8 Statistical analysis

Data with a Gaussian distribution, expressed as the mean and SD, were analyzed using a T test or one-way ANOVA for multiple comparisons. Outcome measurements with a non-Gaussian distribution were expressed as the median and interquartile range (IQR) and were analyzed by the Mann-Whitney U test or Kruskal-Wallis test for multiple comparisons. Categorical variables were compared using the Chi-square or Fisher's exact test as appropriate. Cox-regression hazard analysis assessed the relative risk for arthritis development in subgroups of autoantibody positive arthralgia patients clustering together with RA patients compared to the subgroups of patients who did not cluster together with RA patients. Data were analyzed using the Statistical Package for Social Sciences version 14.0 (SPSS; Chicago, Illinois, United States) and were considered significant with two sided p-values less than 0.05.

### Example 9 Gene expression profiles of ACPA and/or RF positive arthralgia patients

The gene expression profiles of peripheral blood cells derived from 19 arthralgia patients positive for ACPA and/or RF were analyzed and compared to the profiles of 6 ACPA and RF negative healthy controls. A total of 3484 genes were significantly upregulated in the arthralgia group whereas 2829 genes were significantly downregulated compared to healthy controls (false discovery rate (FDR) 5%).

In order to visualize these results in a comprehensive manner, 255 significantly differential genes were selected whose transcript levels were at least twofold differentially expressed between the two groups and unsupervised two-way hierarchical cluster analysis was applied, All autoantibody negative healthy controls concentrated together in one arm of the dendogram. The autoantibody positive arthralgia patients were characterized by differential expression of two gene clusters designated as cluster A, which consisted of genes that are upregulated in the risk group and cluster B with genes that are downregulated compared to the control group.

To interpret the biological function of the genes in clusters A and B, two different pathway-level analysis programs were used: TermFinder and Protein ANalysis THrough Evolutionary Relationships (PANTHER) classification analysis. These analyses look within each gene cluster for significant overrepresentation of genes involved in a biological process. Cluster A contained genes involved in several immune defense related processes: immune (system) response, (cellular) defense response, regulation of the MAPKKK cascade, protein maturation via proteolysis, interferon (IFN) mediated immunity, NK cell mediated immunity and immunity and defense. Cluster B contained genes that are downregulated in most of the persons at risk. These genes could not be classified into a biological process and apparently this cluster contains many genes with unknown function.

To confirm the differential gene expression profiles between autoantibody positive arthralgia patients and controls in a larger cohort we carefully selected 46 genes based on SAM and PAM analysis or biological relevance supplemented with an RA associated set of 20 type I IFN response genes (Table 2). Detailed information on the criteria for gene selection is described above. The expression levels of these genes were measured using TLDA in a larger cohort of autoantibody positive arthralgia patients (n=109) and autoantibody negative healthy controls (n=25). As shown in Table 2, the average expression levels of 25 of the selected genes were significantly differentially expressed between autoantibody positive arthralgia patients and controls, thereby confirming the microarray results. In addition, this analysis showed that the expression levels were highly variable within the autoantibody positive arthralgia patient group (Figure 1). These results show that the expression profiles of autoantibody positive arthralgia patients are clearly distinct from those of healthy controls, especially with respect to increased expression levels of IFN-induced genes.

### Example 10 Heterogeneity of autoantibody positive arthralgia patients

The previous analyses already revealed that not all persons at risk display similar expression profiles. To investigate the molecular heterogeneity within the risk group in more detail, a two-way hierarchical cluster analysis was performed using the microarray data of the autoantibody positive arthralgia patients only (n=19). Therefore, 554 genes were selected whose transcript levels deviated more than twofold from the median expression level in at least four patients. The structure of the dendogram indicated that the autoantibody positive arthralgia patients were separated in subgroups. Indicative for the robustness of sub-classification, application of different gene selection criteria did not alter this result (data not shown).

The position of any autoantibody-positive arhralgia patient in a dendogram was determined by differentially expressed genes that were categorized in four clusters (A, B, C and D). Pathway level analysis on the coordinately regulated genes in each cluster provides a basis for the biological interpretation. Cluster A is characterized by the expression of genes involved in B-cell mediated immunity. Genes involved in macrophage, T-cell, NK-cell and granulocyte mediated immunity were characteristic for cluster B. Cluster C contained genes of yet unknown processes, although some of these genes are known for their role in inflammation (e.g. PBEF1, SSP1 and S100A12). Genes that represent chemokine and cytokine mediated immunity, such as CCL5, IFNg and IL32, were characteristic for cluster D. In aggregate, the two-way hierarchical cluster analysis demonstrated molecular heterogeneity among autoantibody positive arthralgia patients based on differential expression of genes that are involved in diverse arms of the immune response.

To confirm the molecular heterogeneity among arthralgia patients we validated these results in a large cohort of 109 auto-antibody positive arthralgia patients. Therefore, we used 87 genes, consisting of 46 genes most representative for the gene clusters A, B, C and D, which were selected based on PAM, SAM or biological relevance for further analysis using TLDA (Table 3), supplemented with 21 genes (Table 2) that were shown to be differentially expressed within the at-risk group based on the comparison between healthy controls (Figure 1). Two-way hierarchical cluster analysis using all 109 at-risk individuals in combination with the expression profiles of the 87 genes confirmed the existence of heterogeneity among autoantibody positive arthralgia patients.

Moreover, pathway-level analysis revealed that heterogeneity was based on differential expression of clusters of genes involved in essentially the same processes as mentioned earlier, i.e. IFN-mediated immunity, B-cell activation, cytokine/chemokine mediated immunity and some genes with unknown function. The gene cluster involving genes in macrophage, T-cell, NK-cell and granulocyte mediated immunity was not prominently present in this cluster diagram. Instead, the theme "hematopoiesis" was represented.

This analysis confirmed the molecular heterogeneity between autoantibody positive arthralgia patients based on the differential expression of genes reflecting skewed immune processes.

### Example 11 Comparative analysis of blood gene expression profiles from autoantibody positive arthralgia patients and patients with RA

Next, we wanted to investigate the existence of commonalities between (subsets of) autoantibody positive arthralgia patients and RA patients with established disease. Therefore, we analyzed the resemblance between the gene expression characteristics of the heterogeneous at risk subgroup and those of RA patients with established disease (n=25) utilizing all 87 genes (Tables 2 and 3). Two-way hierarchical cluster analysis of the autoantibody positive arthralgia patients and RA patients on the basis of these genes divided the patients into essentially similar subgroups as observed for the subclassification of the arthralgia patients only. In order to reduce the gene set that could be used for classification purposes we performed the same cluster analysis using a selected set of 52 genes, consisting of those genes that had the highest variance in the different gene clusters. A two-way hierarchical cluster analysis based on this gene set divided the autoantibody positive arthralgia patients and RA patients into four subgroups. Most remarkable, RA patients with established disease preferentially co-clustered with at risk individuals of groups I and II compared to those in groups III and IV (Fisher's exact test P<0.01). Groups I and II were characterized by an increased expression of genes involved IFN-mediated immunity and hematopoiesis, respectively, whereas groups III and IV are associated with increased expression of genes involved in cytokine/chemokine mediated immunity and B cell activation, respectively.

These data show that increased expression of genes involved IFN-mediated immunity and/or hematopoiesis reflects a common denominator present in both, a subgroup of autoantibody positive arthralgia patients and patients with established RA. Hence the patients in this subgroup may be more likely to develop RA.

### Example 12 Gene expression profiles predict arthritis development, independent of ACPA levels

In order to study the association between the arthralgia subgroups and the development of arthritis we performed an interim analysis to determine the distribution over the different subgroups of those autoantibody positive arthralgia patients who have developed arthritis in the course of the study. Interim analysis revealed that 20 autoantibody positive arthralgia patients have developed arthritis after a median of 7 months (IQR 4-15; median follow-up of all patients is 30 [IQR 22-39] months) in a median of 3 joints (IQR 3-5).

Cox-regression analysis showed that subgroups I and II were associated with arthritis development (Hazard Ratio [HR] 5.1; 95% confidence interval [C.I.] 1.2-21.9; P=0.03). Correcting for ACPA decreased the HR to 4.1 (95% C.I. 1.0-17.9; P=0.06), resulting in a strong trend independent of ACPA status, implicating that in the presence of ACPA, gene expression profiles specific for subgroups I and II have predictive value for identifying those patients at risk for the development of RA. The presence of the 'shared epitope' genotype, as well as mean ACPA and RF levels were similar in both arthralgia subgroups (I and II versus III and IV, data not shown) and did not influence these results. Excluding those autoantibody positive arthralgia patients who had received two intramuscular dexamethasone injections (n=25) in a trial of primary prevention of RA did not alter these results.

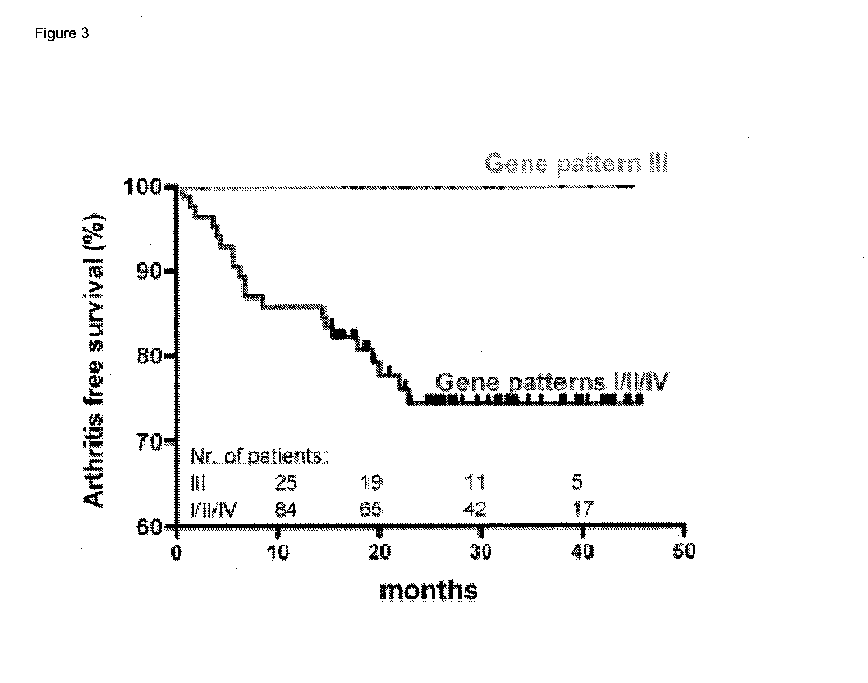

Essentially, similar results were found when excluding the RA patients from the two-way hierarchical cluster analysis. This analysis with auto-antibody arthralgia patients only, resulted in the formation of a subgroup (n=25) without arthralgia patients who have converted to RA. Inclusion of a "dummy" RA converter case in this group, which is required for Cox regression analysis resulted in borderline significance (Hazard Ratio [HR] 7.0; 95% confidence interval [C.I.] 0.94-52.2; P=0.057; Figure 3).

These analyses reveal that autoantibody positive arthralgia patients with high expression of genes involved in IFN mediated immunity, cytokine/chemokine mediated immunity, or hematopoiesis are more likely to develop arthritis. Conversely, autoantibody positive arthralgia patients with high expression of genes involved in B-cell mediated immunity may be protected against development of arthritis or progression towards disease pathogenesis may be suppressed.

### Example 13 Discussion

In this document we demonstrate the heterogeneous nature of ACPA+ and/or RF+ arthralgia patients at risk for development of RA. We identified a set of genes whose expression profiles segregate arthralgia patients at risk for RA into four different groups. Most interestingly, the group of patients that is characterized by increased expression of genes involved in humoral immunity are devoid of cases who have developed arthrtitis in the follow-up period. Subgroups that are characterized by a gene signature of IFN- mediated immunity, cytokine and chemokine activity, and haematopoiesis all contain at risk persons who have developed arthritis. Our results indicate that predisposition for the development of arthritis can already be observed in the peripheral blood gene expression profile of ACPA+ and/or RF+ individuals at risk. Thus, the gene set that distinguishes autoantibody positive arthralgia patients can be used to predict the diagnosis in preclinical RA. This signature can be used to decide on preventive treatment for the development of RA. Moreover, genes that are associated with development of RA are potential targets for the rational to development of new arthritis drugs. The presence of these gene expression characteristics increases the risk for arthritis development ∼5-fold. These results may provide insight in the pathogenic mechanism leading to development of RA and are indicative for an additional biomarkers (set) for the detection of individuals at risk for the development of rheumatoid arthritis.

The functional annotation for the genes provides insight into the underlying biological mechanism leading to progression to and prevention of arthritis. Genes involved IFN- mediated immunity, cytokine and chemokine activity, and haematopesis with the in B-cell immunology are significantly upregulated in the preventive signature

In this study we performed a comparative analysis of the individuals at risk for RA and patients with established RA. Patients with established RA exhibit similar expression patterns to the autoantibody positive arthralgia patients who cluster in the groups containing arthritis follow-up cases. We conclude from this comparison that changes in the peripheral blood transcript level that are characteristic for established RA are already present in the asymptomatic phase of disease.

The different gene expression profiles provide evidence for the assignment of at least four subgroups of persons at risk that display highly distinct gene expression profiles reflecting pronounced differences in the immune status. Previously, we have shown molecular heterogeneity of RA patients based on differential expression of type I IFN induced genes [23]. In the present study we could not only confirm this sub-classification of RA patients with established disease, but we could also classify the autoantibody positive arthralgia patients who later developed arthritis into an IFN-high and IFN-low group. The IFN-low group of RA and autoantibody positive arthralgia patients (group II) was characterized by increased expression levels of genes involved in hematopoiesis. The IFN-high RA patients had a significantly higher mean age, a longer disease duration and a higher mean sedimentation rate, but did not differ with respect to disease activity score, CRP levels, RF and ACPA levels compared to the IFN-low RA patients (data not shown). However, the autoantibody positive arthralgia patients in the different subgroups did not differ in mean age, ACPA, IgM-RF and CRP levels (data not shown). Therefore, these two different molecular profiles - IFN and hematopoiesis - separating the RA patients may represent two different mechanisms involved in disease pathogenesis.

Previously, several research groups, including our own, have shown increased expression levels of IFN related genes in different chronic autoimmune diseases such as RA [23], SLE [33], SSc [34], myositis [35] and MS [25]. Whereas in SLE type I IFN is associated with disease severity, in MS it is used as a treatment strategy with partial beneficial effects [36]. These opposing effects of type I IFN in autoimmunity are highly intriguing and suggest that the in vivo interplay between type I IFN and other inflammatory cytokines may determine the balance between protective immunity and autoimmunity [37]. From the current study it is tempting to speculate that type I IFN is involved in inducing disease, although several studies have demonstrated also a possible protective role for type I IFN in RA [38,39]. Type I IFNs are known to upregulate MHC expression and to induce differentiation of monocytes into antigen presenting dendritic cells (DCs) [40,41]. In addition, since immature DCs control peripheral tolerance by deletion of circulating autoreactive T cells, continued type I IFN induced maturation of DCs may lead to a break of peripheral tolerance through activation of autoreactive cells resulting in immunity to self-antigens. Accordingly, a recent study showed that IFN induced protein IFIT4 might play a role in promoting monocyte differentiation into DC-like cells and subsequent regulation of Th1 cell differentiation [42]. These properties of type I IFN may contribute to the initiation of arthritis in those autoantibody positive arthralgia patients who display increased levels of IFN induced genes.

Hence, whereas ACPA/RF seems involved in the development of arthritis the concomitant activation of the type I IFN system might contribute by facilitating a break of tolerance that triggers the process of autoimmunity leading to chronic inflammation.

The group of genes classified as being involved in hematopoiesis contains two genes (KLF1 and ERAF) involved in erythrocyte development, whereas two other genes (BCL2L1 and BAG1) have anti-apoptotic capacities. Interestingly, the gene DARC encodes for an antigen receptor for chemokines and is present on the surface of venular endothelial cells, cerebral neurons and erythrocytes of Duffy antigen positive individuals [43]. On erythrocytes, DARC is described to act as a sink for free pro-inflammatory chemokines present in the bloodstream in order to limit dissemination through blood into other organs and tissues as well as to prevent leukocyte desensitization [44]. A temporal expression pattern was shown for DARC in the endothelial venules of inflamed synovium with increased expression in the early stage of RA and lower expression in patients with prolonged disease [45]. Moreover, the expression of DARC on endothelial cells was essential for the recruitment of neutrophils in a multicellular model of RA synovium [46]. Our results in peripheral blood extend these findings in inflamed synovium and suggest that DARC expression in both the tissue as well as in the peripheral blood compartment plays a functional role in leukocyte migration into the synovium. How these genes are related to disease pathogenesis in combination with the others in the gene cluster (RBM38, TESC and SELENBP1) remains to be established.

Another group of autoantibody positive arthralgia patients revealed abundant expression of genes indicative for alterations in the humoral immunity i.e. B-cell mediated immunity. Both CD79A and CS79B are necessary for the expression and function of the B-cell antigen receptor and MS4A1 encodes for the B-cell antigen CD20. Whereas CD19 is involved in activating the B-cell receptor, FCRL5 inhibits B-cell activation [47]. This subgroup of autoantibody positive arthralgia patients clustered together with only one autoantibody negative RA patient and none of the 24 autoantibody positive arthralgia patients developed arthritis so far. This suggests that patients with a relatively high expression of genes involved in B-cell mediated immunity may be protected against developing disease. This is in line with the hypothesis that B-cells can act as regulators of autoimmune pathology and protect against disease [48]. A recent study using an established mouse model showed that B-cell receptor revision occurs in antigen-activated B-cells and requires the function of IL7R [49]. Interestingly, the receptor revision acted as an important mechanism of peripheral tolerance in order to diminish autoimmune humoral responses. In addition, although RA patients with active disease can successfully be treated with B-cell depleting agents [50], the rodent model of collagen-induced arthritis (CIA) has shown that transfer of activated B-cells has protective effects [51,52]. This could mean that the role of B-cells in protecting against or inducing autoimmunity may depend of the phase of disease.

Another explanation for the relative increased expression of genes involved in B-cell activation may be that it reflects the amount of B-cells and possible migration of B-cells from the periphery to affected joints in those autoantibody positive arthralgia patients who will develop arthritis.

The invention therefore provides another method for determining whether an individual has an increased risk of developing RA by determining the amount of B lymphocytes in circulation and comparing that amount with a normal value and concluding that an individual has an increased risk of developing RA if the amount in said individual is decreased in comparison to the normal value.

The amount of B-cells in circulation may be determined by conventional techniques employing specific B-cell markers but also by using secretion products such as immunoglobulines.

The last subgroup of autoantibody positive arthralgia patients showed evidence for increased cytokine and chemokine mediated immunity especially related to NK-cells and apoptosis. This data is partially in line with a previous study wherein early rheumatoid arthritis patients showed increased blood levels of pro-inflammatory cytokines associated with autoantibodies targeting citrullinated antigens [53]. On the other hand, using a prospective cohort of Norwegian blood donors, Jorgensen and co-workers showed that cytokines and cytokine related markers (i.e. several interleukins, TNF and IFNg) are generally negative in case sera obtained more than 5 years before the diagnosis of RA [11]. Only TNF was significantly increased in case sera less than 5 years before diagnosis compared to controls. Most other cytokines were only significantly increased after the diagnosis of RA, suggesting that these markers appear to be upregulated rather late in RA development. In our study, the identified genes involved in cytokine mediated immunity are, except for IFNg, distinct from the cytokines measured in the previous studies, making a direct comparison impossible. Although this patient group did not cluster together with any RA patient with active disease, two of the autoantibody positive arthralgia patients developed arthritis meaning that these cytokine related genes may be involved in inducing arthritis.

The current study demonstrates that especially ACPA and/or IgM positive arthralgia patients who display increased expression levels of genes involved in IFN-mediated immunity or hematopoiesis have an increased risk for development of arthritis.

An additional set of genes that may be used in a method according to the invention is provided in tables 9 and 10. The expression of the genes presented therein were found to be increased in patients who developed symptoms of RA, some even after a considerable amount of time, even up to 5 or 10 years (Table 9). The expression of the genes shown in table 10 were decreased in patients that did not develop symptoms of RA in the same amount of time.

The genes that serve a role as classifiers between patients who developed RA and those individuals that did not develop RA based on significance analysis using Prediction Analysis of Microarrays (PAM) (Tibshirani R. et al., Diagnosis of multiple cancer types by shrunken centroids of gene expression. Proc. Natl. Acad. Sci. USA 2002, 99: 6567-6572)) are tabulated in Table 11.

### Reference List

1. Mottonen T, Hannonen P, Korpela M, Nissila M, Kautiainen H, Ilonen J, Laasonen L, Kaipiainen-Seppanen O, Franzen P, Helve T, Koski J, Gripenberg-Gahmberg M, Myllykangas-Luosujarvi R, Leirisalo-Repo M (2002) Delay to institution of therapy and induction of remission using single-drug or combination-disease-modifying antirheumatic drug therapy in early rheumatoid arthritis. Arthritis Rheum 46: 894-898.
2. O'Dell JR (2002) Treating rheumatoid arthritis early: a window of opportunity? Arthritis Rheum 46: 283-285.
3. Quinn MA, Conaghan PG, Emery P (2001) The therapeutic approach of early intervention for rheumatoid arthritis: what is the evidence? Rheumatology (Oxford) 40: 1211-1220.
4. Emery P, McInnes IB, van VR, Kraan MC (2008) Clinical identification and treatment of a rapidly progressing disease state in patients with rheumatoid arthritis. Rheumatology (Oxford) 47: 392-398.
5. Goekoop-Ruiterman YP, de Vries-Bouwstra JK, Allaart CF, van ZD, Kerstens PJ, Hazes JM, Zwinderman AH, Peeters AJ, de Jonge-Bok JM, Mallee C, de Beus WM, de Sonnaville PB, Ewals JA, Breedveld FC, Dijkmans BA (2007) Comparison of treatment strategies in early rheumatoid arthritis: a randomized trial. Ann Intern Med 146: 406-415.
6. Nielen MM, van Schaardenburg D, Reesink HW, van de Stadt RJ, van der Horst-Bruinsma IE, de Koning MH, Habibuw MR, Vandenbroucke JP, Dijkmans BA (2004) Specific autoantibodies precede the symptoms of rheumatoid arthritis: a study of serial measurements in blood donors. Arthritis Rheum 50: 380-386.
7. Rantapaa-Dahlqvist S, de Jong BA, Berglin E, Hallmans G, Wadell G, Stenlund H, Sundin U, van Venrooij WJ (2003) Antibodies against cyclic citrullinated peptide and IgA rheumatoid factor predict the development of rheumatoid arthritis. Arthritis Rheum 48: 2741-2749.
8. Aho K, Palosuo T, Raunio V, Puska P, Aromaa A, Salonen JT (1985) When does rheumatoid disease start? Arthritis Rheum 28: 485-489.
9. Aho K, von ER, Kurki P, Palosuo T, Heliovaara M (1993) Antikeratin antibody and antiperinuclear factor as markers for subclinical rheumatoid disease process. J Rheumatol 20: 1278-1281.
10. Aho K, Palosuo T, Heliovaara M, Knekt P, Alha P, von ER (2000) Antifilaggrin antibodies within "normal" range predict rheumatoid arthritis in a linear fashion. J Rheumatol 27: 2743-2746.
11. Jorgensen KT, Wiik A, Pedersen M, Hedegaard CJ, Vestergaard BF, Gislefoss RE, Kvien TK, Wohlfahrt J, Bendtzen K, Frisch M (2008) Cytokines, autoantibodies and viral antibodies in premorbid and postdiagnostic sera from patients with rheumatoid arthritis: case-control study nested in a cohort of Norwegian blood donors. Ann Rheum Dis 67: 860-866.
12. Kuhn KA, Kulik L, Tomooka B, Braschler KJ, Arend WP, Robinson WH, Holers VM (2006) Antibodies against citrullinated proteins enhance tissue injury in experimental autoimmune arthritis. J Clin Invest 116: 961-973.
13. Lundberg K, Nijenhuis S, Vossenaar ER, Palmblad K, van Venrooij WJ, Klareskog L, Zendman AJ, Harris HE (2005) Citrullinated proteins have increased immunogenicity and arthritogenicity and their presence in arthritic joints correlates with disease severity. Arthritis Res Ther 7: R458-R467.
14. Quinn MA, Gough AK, Green MJ, Devlin J, Hensor EM, Greenstein A, Fraser A, Emery P (2006) Anti-CCP antibodies measured at disease onset help identify seronegative rheumatoid arthritis and predict radiological and functional outcome. Rheumatology (Oxford) 45: 478-480.
15. Hill JA, Bell DA, Brintnell W, Yue D, Wehrli B, Jevnikar AM, Lee DM, Hueber W, Robinson WH, Cairns E (2008) Arthritis induced by posttranslationally modified (citrullinated) fibrinogen in DR4-IE transgenic mice. J Exp Med 205: 967-979.
16. Hill JA, Southwood S, Sette A, Jevnikar AM, Bell DA, Cairns E (2003) Cutting edge: the conversion of arginine to citrulline allows for a high-affinity peptide interaction with the rheumatoid arthritis-associated HLA-DRB1*0401 MHC class II molecule. J Immunol 171: 538-541.
17. Wolfgang J.Hueber, Beren H.Tomooka, Kevin Deane, Lezlie A.Parrish, V.Michael Holers, William H.Robinson (2007) Autoantibody Profiling in Pre-Disease RA Samples. Arthritis Rheum
18. Rantapaa-Dahlqvist S, Boman K, Tarkowski A, Hallmans G (2007) Up regulation of monocyte chemoattractant protein-1 expression in anti-citrulline antibody and immunoglobulin M rheumatoid factor positive subjects precedes onset of inflammatory response and development of overt rheumatoid arthritis. Ann Rheum Dis 66: 121-123.
19. Bos WH, Ursum J, de VN, Bartelds GM, Wolbink GJ, Nurmohamed MT, van der Horst-Bruinsma IE, van de Stadt RJ, Crusius JB, Tak PP, Dijkmans BA, van SD (2008) The role of the shared epitope in arthralgia with anti-cyclic citrullinated peptide antibodies (anti-CCP), and its effect on anti-CCP levels. Ann Rheum Dis 67: 1347-1350.
20. van der Heijde DM, van't HM, van Riel PL, van de Putte LB (1993) Development of a disease activity score based on judgment in clinical practice by rheumatologists. J Rheumatol 20: 579-581.
21. Arnett FC, Edworthy SM, Bloch DA, McShane DJ, Fries JF, Cooper NS, Healey LA, Kaplan SR, Liang MH, Luthra HS,. (1988) The American Rheumatism Association 1987 revised criteria for the classification of rheumatoid arthritis. Arthritis Rheum 31: 315-324.
22. Klein F, Janssens MB (1987) Standardisation of serological tests for rheumatoid factor measurement. Ann Rheum Dis 46: 674-680.
23. van der Pouw Kraan TC, Wijbrandts CA, van Baarsen LG, Voskuyl AE, Rustenburg F, Baggen JM, Ibrahim SM, Fero M, Dijkmans BA, Tak PP, Verweij CL (2007) Rheumatoid arthritis subtypes identified by genomic profiling of peripheral blood cells: assignment of a type I interferon signature in a subpopulation of patients. Ann Rheum Dis 66: 1008-1014.
24. Demeter J, Beauheim C, Gollub J, Hemandez-Boussard T, Jin H, Maier D, Matese JC, Nitzberg M, Wymore F, Zachariah ZK, Brown PO, Sherlock G, Ball CA (2007) The Stanford Microarray Database: implementation of new analysis tools and open source release of software. Nucleic Acids Res 35: D766-D770.
25. van Baarsen LG, van der Pouw Kraan TC, Kragt JJ, Baggen JM, Rustenburg F, Hooper T, Meilof JF, Fero MJ, Dijkstra CD, Polman CH, Verweij CL (2006) A subtype of multiple sclerosis defined by an activated immune defense program. Genes Immun 7: 522-531.
26. Tusher VG, Tibshirani R, Chu G (2001) Significance analysis of microarrays applied to the ionizing radiation response. Proc Natl Acad Sci U S A 98: 5116-5121.
27. Eisen MB, Spellman PT, Brown PO, Botstein D (1998) Cluster analysis and display of genome-wide expression patterns. Proc Natl Acad Sci U S A 95: 14863-14868.
28. Thomas PD, Campbell MJ, Kejariwal A, Mi H, Karlak B, Daverman R, Diemer K, Muruganujan A, Narechania A (2003) PANTHER: a library of protein families and subfamilies indexed by function. Genome Res 13: 2129-2141.
29. Thomas PD, Kejariwal A, Guo N, Mi H, Campbell MJ, Muruganujan A, Lazareva-Ulitsky B (2006) Applications for protein sequence-function evolution data: mRNA/protein expression analysis and coding SNP scoring tools. Nucleic Acids Res 34: W645-W650.
30. Ashburner M, Ball CA, Blake JA, Botstein D, Butler H, Cherry JM, Davis AP, Dolinski K, Dwight SS, Eppig JT, Harris MA, Hill DP, Issel-Tarver L, Kasarskis A, Lewis S, Matese JC, Richardson JE, Ringwald M, Rubin GM, Sherlock G (2000) Gene ontology: tool for the unification of biology. The Gene Ontology Consortium. Nat Genet 25: 25-29.
31. Klareskog L, Alfredsson L, Rantapaa-Dahlqvist S, Berglin E, Stolt P, Padyukov L (2004) What precedes development of rheumatoid arthritis? Ann Rheum Dis 63 Suppl 2: ii28-ii31.
32. Klareskog L, Ronnelid J, Lundberg K, Padyukov L, Alfredsson L (2008) Immunity to citrullinated proteins in rheumatoid arthritis. Annu Rev Immunol 26: 651-675.
33. Baechler EC, Batliwalla FM, Karypis G, Gaffney PM, Ortmann WA, Espe KJ, Shark KB, Grande WJ, Hughes KM, Kapur V, Gregersen PK, Behrens TW (2003) Interferon-inducible gene expression signature in peripheral blood cells of patients with severe lupus. Proc Natl Acad Sci U S A 100: 2610-2615.
34. Tan FK, Zhou X, Mayes MD, Gourh P, Guo X, Marcum C, Jin L, Arnett FC, Jr. (2006) Signatures of differentially regulated interferon gene expression and vasculotrophism in the peripheral blood cells of systemic sclerosis patients. Rheumatology (Oxford) 45: 694-702.
35. Walsh RJ, Kong SW, Yao Y, Jallal B, Kiener PA, Pinkus JL, Beggs AH, Amato AA, Greenberg SA (2007) Type I interferon-inducible gene expression in blood is present and reflects disease activity in dermatomyositis and polymyositis. Arthritis Rheum 56: 3784-3792.
36. The IFNB Multiple Sclerosis Study Group and The University of British Columbia MS/MRI Analysis Group (1995) Interferon beta-1b in the treatment of multiple sclerosis: final outcome of the randomized controlled trial. Neurology 45: 1277-1285.
37. Banchereau J, Pascual V, Palucka AK (2004) Autoimmunity through cytokine-induced dendritic cell activation. Immunity 20: 539-550.
38. van Holten J, Reedquist K, Sattonet-Roche P, Smeets TJ, Plater-Zyberk C, Vervoordeldonk MJ, Tak PP (2004) Treatment with recombinant interferon-beta reduces inflammation and slows cartilage destruction in the collagen-induced arthritis model of rheumatoid arthritis. Arthritis Res Ther 6: R239-R249.
39. van Holten J, Pavelka K, Vencovsky J, Stahl H, Rozman B, Genovese M, Kivitz AJ, Alvaro J, Nuki G, Furst DE, Herrero-Beaumont G, McInnes IB, Musikic P, Tak PP (2005) A multicentre, randomised, double blind, placebo controlled phase II study of subcutaneous interferon beta-1 a in the treatment of patients with active rheumatoid arthritis. Ann Rheum Dis 64: 64-69.
40. Luft T, Pang KC, Thomas E, Hertzog P, Hart DN, Trapani J, Cebon J (1998) Type I IFNs enhance the terminal differentiation of dendritic cells. J Immunol 161: 1947-1953.
41. Santini SM, Lapenta C, Logozzi M, Parlato S, Spada M, Di PT, Belardelli F (2000) Type I interferon as a powerful adjuvant for monocyte-derived dendritic cell development and activity in vitro and in Hu-PBL-SCID mice. J Exp Med 191: 1777-1788.
42. Huang X, Shen N, Bao C, Gu Y, Wu L, Chen S (2008) Interferon-induced protein IFIT4 is associated with systemic lupus erythematosus and promotes differentiation of monocytes into DC-like cells. Arthritis Res Ther 10: R91.
43. Hadley TJ, Peiper SC (1997) From malaria to chemokine receptor: the emerging physiologic role of the Duffy blood group antigen. Blood 89: 3077-3091.
44. Pruenster M, Rot A (2006) Throwing light on DARC. Biochem Soc Trans 34: 1005-1008.
45. Gardner L, Wilson C, Patterson AM, Bresnihan B, FitzGerald O, Stone MA, Ashton BA, Middleton J (2006) Temporal expression pattern of Duffy antigen in rheumatoid arthritis: up-regulation in early disease. Arthritis Rheum 54: 2022-2026.
46. Smith E, McGettrick HM, Stone MA, Shaw JS, Middleton J, Nash GB, Buckley CD, Ed RG (2008) Duffy antigen receptor for chemokines and CXCL5 are essential for the recruitment of neutrophils in a multicellular model of rheumatoid arthritis synovium. Arthritis Rheum 58: 1968-1973.
47. Haga CL, Ehrhardt GR, Boohaker RJ, Davis RS, Cooper MD (2007) Fc receptor-like 5 inhibits B cell activation via SHP-1 tyrosine phosphatase recruitment. Proc Natl Acad Sci U S A 104: 9770-9775.
48. Fillatreau S, Gray D, Anderton SM (2008) Not always the bad guys: B cells as regulators of autoimmune pathology. Nat Rev Immunol 8: 391-397.
49. Wang YH, Diamond B (2008) B cell receptor revision diminishes the autoreactive B cell response after antigen activation in mice. J Clin Invest 118: 2896-2907.
50. Edwards JC, Szczepanski L, Szechinski J, Filipowicz-Sosnowska A, Emery P, Close DR, Stevens RM, Shaw T (2004) Efficacy of B-cell-targeted therapy with rituximab in patients with rheumatoid arthritis. N Engl J Med 350: 2572-2581.
51. Mauri C, Gray D, Mushtaq N, Londei M (2003) Prevention of arthritis by interleukin 10-producing B cells. J Exp Med 197: 489-501.
52. Evans JG, Chavez-Rueda KA, Eddaoudi A, Meyer-Bahlburg A, Rawlings DJ, Ehrenstein MR, Mauri C (2007) Novel suppressive function of transitional 2 B cells in experimental arthritis. J Immunol 178: 7868-7878.
53. Hueber W, Kidd BA, Tomooka BH, Lee BJ, Bruce B, Fries JF, Sonderstrup G, Monach P, Drijfhout JW, van Venrooij WJ, Utz PJ, Genovese MC, Robinson WH (2005) Antigen microarray profiling of autoantibodies in rheumatoid arthritis. Arthritis Rheum 52: 2645-2655.

### TABLES

**Table 1. Study population**

| | controls | arthralgia patients | RA patients* |
|---|---|---|---|
| Total (n) | 25 | 109 | 25 |
| Age in years mean ± SD | 46 ± 13 | 49 ± 10 | 58 ± 12 |
| Female (%) | 17 (68) | 75 (69) | 19 (76) |
| ACPA positive, IgM-RF | - | 37 (34) | 7 (28) |
| ACPA negative, IgM-RF | - | 39 (36) | 0 (0) |
| ACPA and IgM-RF positive | - | 33 (30) | 13 (52) |

| | | | |
|---|---|---|---|
| RA=rheumatoid arthritis, SD=standard deviation, IQR= interquartile range, ACPA=anti-citrullinated protein antibodies, IgM-RF = IgM rheumatoid factor * Five RA patients were ACPA and RF negative | | | |

**Table 2. Genes selected for validation of observed differences in gene expression profiles between autoantibody positive arthralgia patients and healthv controls.**

| **Gene Symbol** | **Full name** | **Reason^{†}** | **Risk *vs.* HD (p-value)^{‡}** |
|---|---|---|---|
| C12orf35 | chromosome 12 open reading frame 35 | 1 | 0.042 |
| C18orf17 | chromosome 18 open reading frame 17 | 3 | 0.001 |
| CCT4 | chaperonin containing TCP1, subunit 4 (delta) | 1 | ns |
| CD274 | CD274 molecule | 3 | 0.009 |
| CKS1B | CDC28 protein kinase regulatory subunit 1 B | 1+2 | ns |
| DDX5 | DEAD (Asp-Glu-Ala-Asp) box polypeptide 5 | 1 | 1.31E-04 |
| DTX3L | deltex 3-like (Drosophila) | 2 | 1.14E-05 |
| EEF1G | eukaryotic translation elongation factor 1 gamma | 3 | ns |
| EIF2AK2 =PKR | eukaryotic translation initiation factor 2-alpha kinase 2 | 3 | 0.001 |
| EPSTI1 | epithelial stromal interaction 1 (breast) | 2+3 | 2.28E-04 |
| FCGR1A | Fc fragment of IgG, high affinity Ia, receptor (CD64) | 3 | 2.00E-05 |
| FLJ31033 | hypothetical protein FLJ31033 | 3 | 1.38E-05 |
| GAPDH | glyceraldehyde-3-phosphate dehydrogenase | Control | ns |
| GBP1 | guanylate binding protein 1, interferon-inducible, 67kDa | 3 | 1.53E-03 |
| HERC3 | hect domain and RLD 3 | 1 | 2.13E-04 |
| HLA-G* | histocompatibility antigen, class I, G | 4 | ND |
| ID1 | inhibitor of DNA binding 1, dominant negative helix-loop-helix protein | 1 | ns |
| IFI44L | interferon-induced protein 44-like | 3 | ns |
| IFI6 | interferon, alpha-inducible protein 6 | 3 | ns |
| IFIH1 =MDA5 | interferon induced with helicase C domain 1 | 2 | 4.41E-05 |
| IFIT1 | interferon-induced protein with tetratricopeptide repeats 1 | 2 | 0.014 |
| IFIT2 | interferon-induced protein with tetratricopeptide repeats 2 | 3 | ns |
| IFITM1 | interferon induced transmembrane protein 1 (9-27) | 3 | 9.13E-04 |
| IFNA2 | interferon, alpha 2 | IFN | ns |
| IFNB1 | interferon, beta 1, fibroblast | IFN | ns |
| IFNG | interferon, gamma | IFN | ns |
| IPO7 | importin 7 | 1 | 0.031 |
| IRF2 | interferon regulatory factor 2 | 3 | 0.013 |
| ISG15 | ISG15 ubiquitin-like modifier | 3 | 0.036 |
| KDELR3* | KDEL (Lys-Asp-Glu-Leu) endoplasmic reticulum protein retention receptor 3 | 4 | ND |
| MAT2B | methionine adenosyltransferase II, beta | 1 | ns |
| MX1 | myxovirus (influenza virus) resistance 1, interferon-inducible protein p78 (mouse) | 3 | ns |
| OAS1 | oligoadenylate synthetase 1, 40/46 kDa | 3 | ns |
| OAS2 | oligoadenylate synthetase 2, 69/71 kDa | 3 | 0.043 |
| OAS3 | oligoadenylate synthetase 3, 100 kDa | 3 | 0.002 |
| PARP14 | poly (ADP-ribose) polymerase family, member 14 | 2+3 | ns |
| PLSCR1 | phospholipid scramblase 1 | 3 | 1.46E-03 |
| REN* | renin | 1 | ND |
| RSAD2 | radical S-adenosyl methionine domain containing 2 | 2 | ns |
| RUFY1 | RUN and FYVE domain containing 1 | 1 | ns |
| SAMD9L | sterile alpha motif domain containing 9-like | 2+3 | ns |
| SELL | selectin L (lymphocyte adhesion molecule 1) | 1 | 0.023 |
| SERPING1 | serpin peptidase inhibitor, clade G (C1 inhibitor), member 1, (angioedema, hereditary) | 3 | 0.008 |
| STAT1 | signal transducer and activator of transcription 1, 91 kDa | 2+3 | 0.041 |
| TNFSF10 =TRAIL | tumor necrosis factor (ligand) superfamily, member 10 | 4 | 4.67E-04 |
| TPM3 | tropomyosin 3 | 1 | ns |
| TRIM22 | tripartite motif-containing 22 | 3 | 5.30E-04 |
| * Gene expression was below detection limit of TLDA analysis | | | |
| † Reason for selection (See M&M for details): 1. PAM analysis: 6 controls vs. 19 persons at risk; 2. PAM analysis: 6 controls vs. 6 ACPA+/RF- at risk; 3. Active RA vs. healthy controls: IFN-induced genes [23]; 4. Explorative SAM analyses between subgroups of patients; IFN: type I/II IFN specific gene; ND: Not detected; NA: Not applicable; ns: not significant; control: gene used for normalization | | | |
| ‡ PCR amplification failed in five cDNA samples (2 controls, 2 autoantibody positive arthralgia patients and 1 RA patient). | | | |

**Table 3 Genes selected for validation of observed heterogeneity in gene expression profiles among autoantibody positive arthralgia patients.**

| **Gene Symbol** | **Full Name** | **Cluster** | **Used analysis** |
|---|---|---|---|
| BAG1 | BCL2-associated athanogene | D | SAM |
| BCL2L1 | BCL2-like 1 | D | PAM |
| CCL5 | chemokine (C-C motif) ligand 5 | B | PAM |
| CD19 | CD19 molecule | A | PAM |
| CD79A | CD79a molecule | A | PAM |
| CD79B | CD79b molecule | A | PAM |
| DARC | Duffy blood group | B | PAM |
| DEFA3 | defensin alpha 3 | D | SAM |
| ERAF | erythroid associated factor | D | PAM |
| FCGR1A† | Fc fragment of IgG, high affinity Ia, receptor (CD64) | B | PAM |
| FCRL5 | Fc receptor-like 5 | A | PAM |
| FLT3LG | fms-related tyrosine kinase 3 ligand | A | Function |
| GADD45B | growth arrest and DNA-damage-inducible beta | A | Function |
| GZMH | granzyme H | B | PAM |
| HDAC10 | histone deacetylase 10 | Group III | SAM |
| IFI27 | interferon, alpha-inducible protein 27 | B | PAM |
| IFNAR2 | interferon (alpha beta and omega) receptor 2 | C | Function |
| IGKC | immunoglobulin kappa constant, immunoglobulin kappa variable 1-5 | A | PAM |
| IGLL1* | immunoglobulin lambda-like polypeptide 1 | A | PAM |
| IL1A* | interleukin 1 alpha | D | Function |
| IL32 | interleukin 32 | A | Function |
| IL7R | interleukin 7 receptor | A | Function |
| KLF1 | Kruppel-like factor 1 (erythroid) | D | PAM |
| LGALS3BP | lectin, galactoside-binding, soluble, 3 binding protein | A | Function |
| LTB | lymphotoxin beta (TNF superfamily, member 3) | D | Function |
| LTF | lactotransferrin | D | PAM |
| MMP9 | matrix metallopeptidase 9 (gelatinase B, 92kDa) | B | PAM |
| MRPL38 | mitochondrial ribosomal protein L38 | C | PAM |
| MS4A1 | membrane-spanning 4-domains, subfamily A, member 1 | A | PAM |
| MYOM2* | myomesin (M-protein) 2, 165kDa | A | SAM |
| NKG7 | natural killer cell group 7 sequence | B | PAM |
| PADI2 | peptidyl arginine deiminase, type II | D | Function |
| PBEF1 | pre-B-cell colony enhancing factor 1 | B | Function |
| PDK3 | pyruvate dehydrogenase kinase isozyme 3 | D | PAM |
| RBM38 | RNA binding motif protein 38 | D | PAM |
| RGS18 | regulator of G-protein signaling 18 | C | SAM |
| RPL23 | ribosomal protein L23 | B | PAM |
| S100A12 | S100 calcium binding protein A12 | B | Function |
| S100A8 | S100 calcium binding protein A8 | B | Function |
| SELENBP1 | selenium binding protein 1 | D | PAM |
| SERPING1† | serpin peptidase inhibitor, clade G (C1 inhibitor), member 1, (angioedema, hereditary) | B | PAM |
| SLC25A1 | solute carrier family 25 (mitochondrial carrier; citrate transporter), member 1 | C | PAM |
| SPP1 | secreted phosphoprotein 1 (osteopontin) | D | Function |
| STAT4 | signal transducer and activator of transcription 4 | B | Function |
| TESC | tescalcin | D | SAM |
| TNFSF7 | CD70 molecule | D | SAM |
| TRGV9 | TCR gamma alternate reading frame protein, T cell receptor gamma variable 9 | B | PAM |
| XRCC5 | X-ray repair complementing defective repair in Chinese hamster cells 5 (double-strand-break rejoining; Ku autoantigen | C | SAM |

| | | | |
|---|---|---|---|
| * gene expression below detection limit of TLDA analysis and therefore excluded from analyses † also present in gene selection based on comparison of autoantibody positive arthralgia patients and healthy controls (Table 2) | | | |

**Table 4: List of differentially expressed genes involved in IFN-mediated immunity**

| **Gene symbol (alias)** | **Gene name** | **Cluster (only Risk)** | **Cytoband** | **Protein location** |
|---|---|---|---|---|
| **EPSTI1** | **Epithelial stromal interaction 1 (breast)** | **IFN** | **13q13.3** | **Intracellular** |
| **IFI6 (G1P3; IFI616)** | **Interferon, alpha-inducible protein 6** | | **1p35** | **Intracellular** |
| **ISG15 (G1P2; IFI15)** | **ISG15 ubiquitinlike modifier** | | **1 p36.33** | **cytoplasma and secreted** |
| **OAS3 (p100)** | **2'5'oligoadenylate synthetase 3, 100kDa** | | **12q24.2** | **Intracellular** |
| **IFI44L** | **Interferoninduced protein 44like** | | **1p31.1** | **Intracellular** |
| **RSAD2 (VIPERIN)** | **Radical Sadenosyl methionine domain containing 2** | | **2p25.2** | **Intracellular** |
| **IFIT1 (G10P1; IFI56; RNM561)** | **Interferoninduced protein with tetratricopeptide repeats 1** | | **10q25-q26** | **membrane** |
| **MX1 (IFI78; MxA)** | **Myxovirus (influenza virus) resistance 1, interferoninducible protein p78 (mouse)** | | **21 q22.3** | **Intracellular** |
| **CD274 (B7H1; PDCD1L1; PDCD1LG1; PDL1)** | **CD274 molecule** | | **9p24** | **Membrane** |
| **SERPING1 (C1-INH; C1INH; C1 NH; HAE1; HAE2)** | **Serpin peptidase inhibitor, clade G (C1 inhibitor), member 1, (angioedema, hereditary)** | | **11q12-q131** | **secreted.** |
| **IFI27 (ISG12A)** | **Interferon, alphainducible protein 27** | | **14q32** | **Membrane** |
| DEFA3;LOC728358; DEFA1 | Defensin, alpha 3, neutrophilspecific | | 8pter-p233 | secreted. |
| LTF | Lactotransferrin | | 3p21.31 | Secreted |
| CKS1B (PNAS-143; PNAS-16; PNAS-18; ckshs1) | CDC28 protein kinase regulatory subunit 1B | IFN | 1 q21.2 | Intracellular |
| TNFSF10 (APO2L; TRAIL) | Tumor necrosis factor (ligand) superfamily, member 10 | | 3q26 | Membrane |
| GADD45B | Growth arrest and DNAdamageinducible, beta | | 19p13.3 | Intracellular |

**Table 5: List of differentially expressed genes involved in B-cell mediated immunity**

| **Gene symbol (alias)** | **Gene name** | **Cluster (only Risk)** | **Cytoband** | **Protein location** |
|---|---|---|---|---|
| **CD19 (B4; MGC12802)** | **CD19 molecule** | **B-cell** | **16p11.2** | **membrane** |
| **CD79A (IGA)** | **CD79a molecule, immunoglobulinassociated alpha** | | **19q13.2** | **cell membrane** |
| **CD79B (IGB)** | **CD79b molecule, immunoglobulinassociated beta** | | **17q23** | **cell membrane** |
| **MS4A1 (CD20; Bp35; LEU-16; MGC3969; MS4A2; S7)** | **Membranespanning 4domains, subfamily A, member 1** | | **11q12** | **membrane** |
| **FCRL5 (BXMAS1; IRTA2)** | **Fc receptorlike 5** | | **1q21** | **membrane and secreted** |
| LTB (TNFC) | Lymphotoxin beta (TNF superfamily, member 3) | | 6p21.3 | membrane |
| MRPL38 | Mitochondrial ribosomal protein L38 | | 17q25.3 | intracellular |
| HDAC10 | Histone deacetylase 10 | | 22q13.31 | intracellular |
| SLC25A1 (CTP) | Solute carrier family 25 (mitochondrial carrier; citrate transporter), member 1 | | 22q11.21 | intracellular |
| RPL23 | Ribosomal protein L23 | | 17q | intracellular |

**Table 6: List of differentially expressed genes involved in hematopoiesis**

| **Gene symbol (alias)** | **Gene name** | **Cluster (only Risk)** | **Cytoband** | **Protein location** |
|---|---|---|---|---|
| **DARC (CCBP1; GPD)** | **Duffy blood group, chemokine receptor** | **Hematopoiesis** | **1 q21-q22** | **membrane** |
| **BCL2L1 (BCL-XL/S; BCLXL; BCLXS; DKFZp781P2092; bcl-xS)** | **BCL2like 1** | | **20q11.21** | **intracellular** |
| **RBM38 (RNPC1)** | **RNA binding motif protein 38** | | **20q13.31** | **intracellular** |
| **BAG1 (RAP46)** | **BCL2associated athanogene** | | **9p12** | **intracellular** |
| **TESC (TSC)** | **Tescalcin** | | **12q24.22** | **intracellular** |
| **KLF1 (EKLF)** | **Kruppellike factor 1 (erythroid)** | | **19p13.13-p13.12** | **intracellular** |
| **ERAF (AHSP; EDRF)** | **Erythroid associated factor** | | **16p11.2** | **Secreted** |
| **SELENBP1 (SP56)** | **Selenium binding protein 1** | | **1q21-q22** | **membrane and cytoplasm** |
| S100A12 (CAAF1; CGRP; ENRAGE; p6) | S100 calcium binding protein A12 | | 1q21 | intracellular |
| S100A8 (CAGA; CALPROTECTIN; CFAG) | S100 calcium binding protein A8 | | 1q21 | Secreted and cytoplasm |

**Table 7: List of differentially expressed genes involved in cytokine mediated immunity**

| **Gene symbol (alias)** | **Gene name** | **Cluster (only Risk)** | **Cytoban d** | **Protein location** |
|---|---|---|---|---|
| **CCL5 (SCYA5; TCP228)** | **Chemokine (CC motif) ligand 5** | **Cytokines** | **17q11.2-q12** | **Secreted** |
| **IFNG** | **Interferon, gamma** | | **12q14** | **Secreted** |
| **GZMH (CCP-X; CGL-2; CGL2; CSP-C; CTLA1; CTSGL2)** | **Granzyme H (cathepsin Glike 2, protein hCCPX)** | | **14q11.2** | **intracellular** |
| **NKG7 (GIG1)** | **Natural killer cell group 7 sequence** | | **19q13.33** | **membrane** |
| DDX5 (DKFZp686J011 90; G17P1; HLR1; HUMP68) | DEAD (AspGluAlaAsp) box polypeptide 5 | | 17q21 | intracellular |
| IPO7 | Importin 7 | | 11p15.4 | intracellular |
| C18orf17 | Chromosome 18 open reading frame 17 | | 18q11.2 | |
| IL7R (CD127; IL7R-ALPHA) | Interleukin 7 receptor | | 5p13 | membrane and secreted |
| STAT4 | Signal transducer and activator of transcription 4 | | 2q32.2-q32.3 | intracellular |

**Table 8. Gene-set predictive for future development of arthritis**

| **Gene symbol (alias)** | **Gene name** | **Cluster (only Risk)** | **Cytoba nd** | **Protein location** |
|---|---|---|---|---|
| **EPSTI1** | **Epithelial stromal interaction 1 (breast)** | **IFN** | **13q13.3** | **Intracellular** |
| **IFI6 (G1 P3; IFI616)** | **Interferon, alpha-inducible protein 6** | | **1p35** | **Intracellular** |
| **ISG15 (G1P2; IFI15)** | **ISG15 ubiquitinlike modifier** | | **1 p36.33** | **cytoplasma and secreted** |
| **OAS3 (p100)** | **2'5'oligoadenylate synthetase 3,100kDa** | | **12q24.2** | **Intracellular** |
| **IFI44L** | **Interferoninduced protein 44like** | | **1p31.1** | **Intracellular** |
| **RSAD2 (VIPERIN)** | **Radical Sadenosyl methionine domain containing 2** | | **2p25.2** | **Intracellular** |
| **IFIT1 (G10P1; IFI56; RNM561)** | **Interferoninduced protein with tetratricopeptide repeats 1** | | **10q25-q26** | **membrane** |
| **MX1 (IFI78; MxA)** | **Myxovirus (influenza virus) resistance 1, interferoninducible protein p78 (mouse)** | | **21q22.3** | **Intracellular** |
| **CD274 (B7H1; PDCD1L1; PDCD1 LG1; PDL1)** | **CD274 molecule** | | **9p24** | **Membrane** |
| **SERPING1 (C1-INH; C1INH; C1NH; HAE1; HAE2)** | **Serpin peptidase inhibitor, clade G (C1 inhibitor), member 1, (angioedema, hereditary)** | | **11q12-q131** | **secreted.** |
| **IFI27 (ISG12A)** | **Interferon, alphainducible protein 27** | | **14q32** | **Membrane** |
| DEFA3;LOC7283 58; DEFA1 | Defensin, alpha 3, neutrophilspecific | | 8pter-p233 | secreted. |
| LTF | Lactotransferrin | | 3p21.31 | Secreted |
| CKS1B (PNAS-143; PNAS-16; PNAS-18; ckshs1) | CDC28 protein kinase regulatory subunit 1 B | IFN | 1q21.2 | Intracellular |
| TNFSF10 (APO2L; TRAIL) | Tumor necrosis factor (ligand) superfamily, member 10 | | 3q26 | Membrane |
| GADD45B | Growth arrest and DNAdamageinducible, beta | | 19p13.3 | Intracellular |
| **CD19 (B4; MGC12802)** | **CD19 molecule** | **B-cell** | **16p11.2** | **membrane** |
| **CD79A (IGA)** | **CD79a molecule, immunoglobulinassociate d alpha** | | **19q13.2** | **cell membrane** |
| **CD79B (IGB)** | **CD79b molecule, immunoglobulinassociate d beta** | | **17q23** | **cell membrane** |
| **MS4A1 (CD20; Bp35; LEU-16; MGC3969; MS4A2; S7)** | **Membranespanning 4domains, subfamily A, member 1** | | **11q12** | **membrane** |
| **FCRL5 (BXMAS1; IRTA2)** | **Fc receptorlike 5** | | **1q21** | **membrane and secreted** |
| LTB (TNFC) | Lymphotoxin beta (TNF superfamily, member 3) | | 6p21.3 | membrane |
| MRPL38 | Mitochondrial ribosomal protein L38 | **B-cell** | 17q25.3 | intracellular |
| HDAC10 | Histone deacetylase 10 | | 22q13.31 | intracellular |
| SLC25A1(CTP) | Solute carrier family 25 (mitochondrial carrier; citrate transporter), member 1 | | 22q11.21 | intracellular |
| RPL23 | Ribosomal protein L23 | | 17q | intracellular |
| **DARC (CCBP1; GPD)** | **Duffy blood group, chemokine receptor** | **Hematopoiesis** | **1q21-q22** | **membrane** |
| **BCL2L1 (BCL-XL/S; BCLXL; BCLXS; DKFZp781P209 2; bcl-xS)** | **BCL2like 1** | | **20q11.21** | **intracellular** |
| **RBM38 (RNPC1)** | **RNA binding motif protein 38** | | **20q13.31** | **intracellular** |
| **BAG1 (RAP46)** | **BCL2associated athanogene** | | **9p12** | **intracellular** |
| **TESC (TSC)** | **Tescalcin** | | **12q24.2 2** | **intracellular** |
| **KLF1 (EKLF)** | **Kruppellike factor 1 (erythroid)** | | **19p13.13-p13.12** | **intracellular** |
| **ERAF (AHSP; EDRF)** | **Erythroid associated factor** | | **16p11.2** | **Secreted** |
| **SELENBP1 (SP56)** | **Selenium binding protein 1** | **Hematopoiesis** | **1q21-q22** | **membrane and cytoplasm** |
| S100A12 (CAAF1; CGRP; ENRAGE; p6) | S100 calcium binding protein A12 | | 1q21 | intracellular |
| S100A8 (CAGA; CALPROTECTIN; CFAG) | S100 calcium binding protein A8 | | 1q21 | Secreted and cytoplasm |
| **CCL5 (SCYA5; TCP228)** | **Chemokine (CC motif) ligand 5** | **Cytokines** | **17q11.2 -q12** | **Secreted** |
| **IFNG** | **Interferon, gamma** | | **12q14** | **Secreted** |
| **GZMH (CCP-X; CGL-2; CGL2; CSP-C; CTLA1; CTSGL2)** | **Granzyme H (cathepsin Glike 2, protein hCCPX)** | | **14q11.2** | **intracellular** |
| **NKG7 (GIG1)** | **Natural killer cell group 7 sequence** | | **19q13.3 3** | **membrane** |
| DDX5 (DKFZp686J011 90; G17P1; HLR1; HUMP68) | DEAD (AspGluAlaAsp) box polypeptide 5 | | 17q21 | intracellular |
| IPO7 | Importin 7 | | 11p15.4 | intracellular |
| C18orf17 | Chromosome 18 open reading frame 17 | | 18q11.2 | |
| IL7R (CD127; IL7R-ALPHA) | Interleukin 7 receptor | | 5p13 | membrane and secreted |
| STAT4 | Signal transducer and activator of transcription 4 | | 2q32.2-q32.3 | intracellular |
| GAPDH | Glyceraldehyde-3-phosphate dehydrogenase | Unknown | 12p13 | intracellular |
| HERC3 | Hect domain and RLD 3 | | 4q21 | Intracellular |
| IRF2 | Interferon regulatory factor 2 | | 4q34.1-q351 | Intracellular |
| XRCC5 (Ku80; Ku86) | Xray repair complementing defective repair in Chinese hamster cells 5 (doublestrandbreak rejoining; Ku autoantigen, 80kDa) | Unknown | 2q35 | Intracellular |
| MAT2B | Methionine adenosyltransferase II, beta | | 5q34-q35 | Intracellular |
| SELL (L-SELECTIN; LAM1;LEU8; LYAM1) | Selectin L (lymphocyte adhesion molecule 1) | | 1 q23-q25 | Membrane |
| TPM3 | Tropomyosin 3 | | 1q21.2 | Intracellular |

**Table S2. TLDA assays**

| **Symbol** | **AssayID** | **Gene Name** |
|---|---|---|
| BAG1 | Hs00185390_m1 | BCL2-associated athanogene |
| BCL2L1 | Hs00169141_m1 | BCL2-like 1 |
| C12orf35 | Hs00216848_m1 | chromosome 12 open reading frame 35 |
| C18orf17 | Hs00400521_m1 | chromosome 18 open reading frame 17 |
| CCL5 | Hs00174575_m1 | chemokine (C-C motif) ligand 5 |
| CCT4 | Hs00272345_m1 | chaperonin containing TCP1, subunit 4 (delta) |
| CD19 | Hs00174333_m1 | CD19 molecule |
| CD274 | Hs00204257_m1 | CD274 molecule |
| CD79A | Hs00998119_m1 | CD79a molecule |
| CD79B | Hs01058826_g1 | CD79b molecule |
| CKS1B | Hs01029137_g1 | CDC28 protein kinase regulatory subunit 1 B |
| DARC | Hs01011079_s1 | Duffy blood group |
| DDX5 | Hs00189323_m1 | DEAD (Asp-Glu-Ala-Asp) box polypeptide 5 |
| DEFA3 | Hs00414018_m1 | defensin alpha 3 |
| DTX3L | Hs00370540_m1 | deltex 3-like (Drosophila) |
| EEF1G | Hs01922638_u1 | eukaryotic translation elongation factor 1 gamma |
| EIF2AK2 =PKR | Hs00169345_m1 | eukaryotic translation initiation factor 2-alpha kinase 2 |
| EPSTI1 | Hs00264424_m1 | epithelial stromal interaction 1 (breast) |
| ERAF | Hs00372339_g1 | erythroid associated factor |
| FCGR1A | Hs00417598_m1 | Fc fragment of IgG, high affinity la, receptor (CD64) |
| FCRL5 | Hs00258709_m1 | Fc receptor-like 5 |
| FLJ31033 | Hs00291459_m1 | hypothetical protein FLJ31033 |
| FLT3LG | Hs00181740_m1 | fms-related tyrosine kinase 3 ligand |
| GADD45B | Hs00169587_m1 | growth arrest and DNA-damage-inducible beta |
| GAPDH | Hs00266705_g1 | glyceraldehyde-3-phosphate dehydrogenase |
| GBP1 | Hs00266717_m1 | guanylate binding protein 1, interferon-inducible, 67kDa |
| GZMH | Hs00277212_m1 | granzyme H |
| HDAC10 | Hs00368899_m1 | histone deacetylase 10 |
| HERC3 | Hs00205934_m1 | hect domain and RLD 3 |
| HLA-G | Hs00365950_g1 | histocompatibility antigen, class I, G |
| ID1 | Hs00357821_g1 | inhibitor of DNA binding 1, dominant negative helix-loop-helix protein |
| IFI27 | Hs00271467_m1 | interferon, alpha-inducible protein 27 |
| IFI44L | Hs00199115_m1 | interferon-induced protein 44-like |
| IFI6 | Hs00242571_m1 | interferon, alpha-inducible protein 6 |
| IFIH1 =MDA5 | Hs00223420_m1 | interferon induced with helicase C domain 1 |
| IFIT1 | Hs01911452_s1 | interferon-induced protein with tetratricopeptide repeats 1 |
| IFIT2 | Hs00533665_m1 | interferon-induced protein with tetratricopeptide repeats 2 |
| IFITM1 | Hs00705137_s1 | interferon induced transmembrane protein 1 (9-27) |
| IFNA2 | Hs00265051_s1 | interferon, alpha 2 |
| IFNAR2 | Hs01022059_m1 | interferon (alpha beta and omega) receptor 2 |
| IFNB1 | Hs01077958_s1 | interferon, beta 1, fibroblast |
| IFNG | Hs00174143_m1 | interferon, gamma |
| IGKC | Hs00415165_m1 | immunoglobulin kappa constant, immunoglobulin kappa variable 1-5 |
| IGLL1 | Hs00252263_m1 | immunoglobulin lambda-like polypeptide 1 |
| IL1A | Hs00174092_m1 | interleukin 1 alpha |
| IL32 | Hs00170403_m1 | interleukin 32 |
| IL7R | Hs00902334_m1 | interleukin 7 receptor |
| IPO7 | Hs00255188_m1 | importin 7 |
| IRF2 | Hs00180006_m1 | interferon regulatory factor 2 |
| ISG15 | Hs00192713_m1 | ISG15 ubiquitin-like modifier |
| KDELR3 | Hs00423556_m1 | KDEL (Lys-Asp-Glu-Leu) endoplasmic reticulum protein retention receptor 3 |
| KLF1 | Hs00610592_m1 | Kruppel-like factor 1 (erythroid) |
| LGALS3BP | Hs00174774_m1 | lectin, galactoside-binding, soluble, 3 binding protein |
| LTB | Hs00242739_m1 | lymphotoxin beta (TNF superfamily, member 3) |
| LTF | Hs00914330_m1 | lactotransferrin |
| MAT2B | Hs00203231_m1 | methionine adenosyltransferase II, beta |
| MMP9 | Hs00234579_m1 | matrix metallopeptidase 9 (gelatinase B, 92kDa) |
| MRPL38 | Hs00375656_m1 | mitochondrial ribosomal protein L38 |
| MS4A1 | Hs00544819_m1 | membrane-spanning 4-domains, subfamily A, member 1 |
| MX1 | Hs00182073_m1 | myxovirus (influenza virus) resistance 1, interferon-inducible protein p78 (mouse) |
| MYOM2 | Hs00187676_m1 | myomesin (M-protein) 2, 165kDa |
| NKG7 | Hs00366585_g1 | natural killer cell group 7 sequence |
| OAS1 | Hs00242943_m1 | oligoadenylate synthetase 1, 40/46 kDa |
| OAS2 | Hs00159719_m1 | oligoadenylate synthetase 2, 69/71 kDa |
| OAS3 | Hs00196324_m1 | oligoadenylate synthetase 3, 100 kDa |
| PADI2 | Hs00247108_m1 | peptidyl arginine deiminase, type II |
| PARP14 | Hs00393814_m1 | poly (ADP-ribose) polymerase family, member 14 |
| PBEF1 | Hs00237184_m1 | pre-B-cell colony enhancing factor 1 |
| PDK3 | Hs00178440_m1 | pyruvate dehydrogenase kinase isozyme 3 |
| PLSCR1 | Hs00275514_m1 | phospholipid scramblase 1 |
| RBM38 | Hs00250139_m1 | RNA binding motif protein 38 |
| REN | Hs00166915_m1 | renin |
| RGS18 | Hs00329468_m1 | regulator of G-protein signaling 18 |
| RPL23 | Hs00745462_s1 | ribosomal protein L23 |
| RSAD2 | Hs00369813_m1 | radical S-adenosyl methionine domain containing 2 |
| RUFY1 | Hs00228528_m1 | RUN and FYVE domain containing 1 |
| S100A12 | Hs00194525_m1 | S100 calcium binding protein A12 |
| S100A8 | Hs00374264_g1 | S100 calcium binding protein A8 |
| SAMD9L | Hs00541567_s1 | sterile alpha motif domain containing 9-like |
| SELENBP1 | Hs00187625_m1 | selenium binding protein 1 |
| SELL | Hs00174151_m1 | selectin L (lymphocyte adhesion molecule 1) |
| SERPING1 | Hs00163781_m1 | serpin peptidase inhibitor, clade G (C1 inhibitor), member 1, (angioedema, hereditary) |
| SLC25A1 | Hs00761590_sH | solute carrier family 25 (mitochondrial carrier; citrate transporter), member 1 |
| SPP1 | Hs00959010_m1 | secreted phosphoprotein 1 (osteopontin) |
| STAT1 | Hs00234829 m1 | signal transducer and activator of transcription 1, 91kDa |
| STAT4 | Hs00231372_m1 | signal transducer and activator of transcription 4 |
| TESC | Hs00215487_m1 | tescalcin |
| TNFSF10 =TRAIL | Hs00234356_m1 | tumor necrosis factor (ligand) superfamily, member 10 |
| TNFSF7 | Hs00174297_m1 | CD70 molecule |
| TPM3 | Hs00383595_m1 | tropomyosin 3 |
| TRGV9 | Hs00233330_m1 | TCR gamma alternate reading frame protein, T cell receptor gamma variable 9 |
| TRI M22 | Hs00232319_m1 | tripartite motif-containing 22 |
| XRCC5 | Hs00221707_m1 | X-ray repair complementing defective repair in Chinese |
| | | hamster cells 5 (double-strand-break rejoining; Ku autoantigen |

**Table 9; The expression of the following genes was found to be increased in individuals who developed Rheumatoid Arthritis.**

| **Name** | **Description** |
|---|---|
| | Homo sapiens translocase of inner mitochondrial membrane 10 homolog |
| LY6E | Homo sapiens lymphocyte antigen 6 complex. locus E mRNA. |
| TRI M22 | Homo sapiens tripartite motif-containing 22 mRNA. |
| SAMD9L | Homo sapiens sterile alpha motif domain containing 9-like mRNA |
| MX1 | Homo sapiens myxovirus (influenza virus) resistance 1. interferon-induc |
| OAS1 | Homo sapiens 2',5'-oligoadenylate svnthetase 1, 40/46kDa . transc |
| OASL | Homo sapiens 2'-5'-oligoadenylate synthetase-like . transcript va |
| OASL | Homo sapiens 2'-5'-oligoadenylate synthetase-like , transcript va |
| EPSTI1 | Homo sapiens epithelial stromal interaction 1 (breast), transc |
| | Homo sapiens interferon-induced protein with tetratricopeptide repeats |
| IFI6 | Homo sapiens interferon. alpha-inducible protein 6 , transcript v |
| IFI44 | Homo sapiens interferon-induced protein 44 , mRNA. |
| ISG15 | Homo sapiens ISG15 ubiquitin-like modifier, mRNA. |
| OAS3 | Homo sapiens 2'-5'-oligoadenylate synthetase 3, 100kDa , mRNA. |
| | Homo sapiens interferon-induced protein with tetratricopeptide repeats |
| HERC5 | Homo sapiens hect domain and RLD 5 , mRNA. |
| RSAD2 | Homo sapiens radical S-adenosyl methionine domain containing 2 , |
| IFI44L | Homo sapiens interferon-induced protein 44-like . mRNA. |
| | Homo sapiens interferon-induced protein with tetratriconeptide repeats |
| XAF1 | Homo sapiens XIAP associated factor 1 , transcript variant 2, mRNA |
| | Homo sapiens signal transducer and activator of transcription 2, 113kDa |
| PARP12 | Homo sapiens poly (ADP-ribose) polymerase family, member 12 , m |
| IFITM3 | Homo sapiens interferon induced transmembrane protein 3 (1-8U) |
| | Homo sapiens eukaryotic translation initiation factor 2-alpha kinase 2 |
| PARP14 | Homo sapiens poly (ADP-ribose) polymerase family, member 14 , m |

**Table 10; The expression of the following genes was found to be decreased in individuals with pre-Rheumatoid Arthritis.**

| **Name** | **Description** |
|---|---|
| | Homo sapiens myocardial infarction associated transcript (non-protein c |
| ITPR3 | Homo sapiens inositol 1,4,5-triphosphate receptor. type 3 , mRNA |
| FAM39E | Homo sapiens family with sequence similarity 39. member E , mRN |
| SI00A4 | Homo sapiens S100 calcium binding protein A4 . transcript varia |
| ARPC | Homo sapiens actin related protein 2/3 complex, subunit 1B, 41 kDa |
| SEC14L1 | Homo sapiens SEC14-like 1 (S. cerevisiae). mRNA. |
| DDX23 | Homo sapiens DEAD (Asp-Glu-Ala-Asp) box -polypeptide 23 , mRNA. |
| | Homo sapiens protein phosphatase 1. catalytic subunit, alpha isoform |
| DIAPH1 | Homo sapiens diaphanous homolog 1 (Drosophila). mRNA. |
| IDH3B | Homo sapiens isocitrate dehydrogenase 3 (NAD+) beta . nuclear ge |
| JAK1 | Homo sapiens Janus kinase 1 (a protein tyrosine kinase). mRNA. |
| DGKA | Homo sapiens diacylglycerol kinase. alpha 80kDa , transcript vari |
| ARHGAP30 | Homo sapiens Rho GTPase activating protein 30 , transcript va |
| MAEA | Homo sapiens macrophage erythroblast attacher, transcript varian |
| PHD | Homo sapiens phosphogluconate dehydrogenase. mRNA. |
| | Homo sapiens dysferlin, limb qirdle muscular dystrophy 2B (autosomal re |
| | Homo sapiens sema domain, immunoglobulin domain (Ig), transmembrane dom |
| | Homo sapiens y-ral simian leukemia viral oncogene homolog B (ras relate |
| | Homo sapiens neutrophil cytosolic factor 1. (chronic granulomatous dise |
| FPR2 | Homo sapiens formvl peptide receptor 2 . transcript variant 1, mR |
| ZNFX1 | Homo sapiens zinc finger, NFX1-type containing 1 . mRNA. |
| ADAR | Homo sapiens adenosine deaminase, RNA-specific , transcript varia |
| CTSA | Homo sapiens cathepsin A , mRNA. |
| PLAUR | Homo sapiens plasminogen activator. urokinase receptor . transcr |
| ALDOA | Homo sapiens aldolase A. fructose-bisphosphate , transcript vari |
| | Homo sapiens phosphoinositide-3-kinase, catalytic. delta polypeptide (P |
| CXCR4 | Homo sapiens chemokine (C-X-C motif) receptor 4 , transcript vat |
| I8RB | Homo sapiens interleukin 8 receptor. beta , mRNA. |
| | Homo sapiens 0-linked N-acetylglucosamine (GlcNAc) transferase (UDP-N-a |
| | Homo sapiens T cell receptor alpha locus. mRNA (cDNA clone MGC: 88342 IM |
| | Homo sapiens full-length cDNA clone CS0CAP005YH21 of Thymus |
| VDAC1 | Homo sapiens voltage-dependent anion channel 1 , mRNA. |
| DNAJA3 | Homo sapiens DnaJ (Hsp40) homolog, subfamily A. member 3 , mRNA |
| PPTC7 | Homo sapiens PTC7 Protein phosphatase homolog (S. cerevisiae), |
| ALPL | Homo sapiens alkaline phosphatase. liver/bone/kidney , mRNA |
| CA4 | Homo sapiens carbonic anhydrase IV . mRNA |
| PGLYRPI | Homo sapiens peptidoglycan recognition protein 1 , mRNA. |
| | Homo sapiens solute carrier family 11 (proton-coupled divalent metal io |
| KIAA1324 | Homo sapiens KIAA1324 , mRNA. |
| PAD14 | Homo sapiens peptidyl arginine deiminase. type IV , mRNA. |
| | Homo sapiens matrix metallopeptidase 9 (gelatinase B, 92kDa gelatinase, |
| CDA | Homo sapiens cvtidine deaminase , mRNA. |
| | Homo sapiens cysteine-rich secretory protein LCCL domain containing 2 ( |
| MY01F | Homo sapiens myosin IF , mRNA |
| MBOA | Homo sapiens membrane bound 0-acyltransferase domain containing 7 ( |
| IL8RA | Homo sapiens interleukin 8 receptor, alpha , mRNA. |

**Table 11 The following genes were found to be differentially expressed in a genome wide analysis.**

| **Name** | **Description** | **Up (+) or downregulated (-) in non_RA patients** | **Up (+) or downregulated (-) in RA patients** |
|---|---|---|---|
| HERC5 | Homo sapiens hect domain and RLD 5 (HERC5), mRNA | -0.1677 | 0.2516 |
| SDPR | Homo sapiens serum deprivation response (phosphatidylserine binding protein) (SDPR), r | -0.1615 | 0.2422 |
| JAK1 | Homo sapiens Janus kinase 1 (a protein tyrosine kinase) (JAK1), mRNA. | 0.1609 | -0.2413 |
| LOC651436 | PREDICTED: Homo sapiens similar to ribosomal protein L9 (LOC651436), mRNA. | -0.153 | 0.2294 |
| DYSF | Homo sapiens dysferlin, limb girdle muscular dystrophy 2B (autosomal recessive) (DYSF), | 0.1487 | -0.2231 |
| RALB | Homo sapiens v-ral simian leukemia viral oncogene homolog B (ras related; GTP binding | 0.1457 | -0.2185 |
| ARHGAP30 | Homo sapiens Rho GTPase activating protein 30 (ARHGAP30), transcript variant 2, mRNA | 0.1435 | -0.2153 |
| RNASE2 | Homo sapiens ribonuclease, RNase A family, 2 (liver, eosinophil-derived neurotoxin) (RNA | -0.1433 | 0.215 |
| no symbol | Homo sapiens T cell receptor alpha locus, mRNA (cDNA clone MGC:88342 IMAGE:30352 | 0.1425 | -0.2137 |
| LOC642250 | Homo sapiens hCG39912 (LOC642250), mRNA. | -0.1416 | 0.2123 |
| RPL17 | Homo sapiens ribosomal protein L17 (RPL17), transcript variant 2, mRNA | -0.1413 | 0.2119 |
| CYP27A1 | Homo sapiens cytochrome P450, family 27, subfamily A, polypeptide 1 (CYP27A1), nuclea | 0.1389 | -0.2083 |
| HES4 | Homo sapiens hairy and enhancer of split 4 (Drosophila) (HES4), mRNA. | -0.1359 | 0.2038 |
| KIAA1324 | Homo sapiens KIAA1324 (KIAA1324), mRNA. | 0.1354 | -0.2031 |
| IF144 | Homo sapiens interferon-induced protein 44 (IF144), mRNA. | -0.1352 | 0.2028 |
| C15orf15 | Homo sapiens chromosome 15 open reading frame 15 (C15orf15), mRNA | -0.1343 | 0.2014 |
| RGS18 | Homo sapiens regulator of G-protein signaling 18 (RGS18), mRNA. | -0.1342 | 0.2013 |
| no symbol | AV737317 CB Homo sapiens cDNA clone CBCAQH03 5, mRNA sequence | 0.1327 | -0.1991 |
| CTSZ | Homo sapiens cathepsin Z (CTSZ), mRNA. | -0.132 | 0.198 |
| EIF2C2 | Homo sapiens eukaryotic translation initiation factor 2C, 2 (EIF2C2), mRNA. | -0.132 | 0.198 |
| CLC | Homo sapiens Charcot-Leyden crystal protein (CLC), mRNA. | -0.1317 | 0.1975 |
| MED25 | Homo sapiens mediator complex subunit 25 (MED25), mRNA. | 0.1314 | -0.1971 |
| LOG651064 | PREDICTED: Homo sapiens hypothetical protein LOC651064 (LOC651064), mRNA. | -0.1313 | 0.1969 |
| LOC650518 | PREDICTED: Homo sapiens similar to Proteasome subunit alpha type 6 (Proteasome iota | -0.131 | 0.1965 |
| PPP1CA | Homo sapiens protein phosphatase 1, catalytic subunit, alpha isoform (PPP1 CA), transcrip | 0.1308 | -0.1962 |
| ALDOA | Homo sapiens aldolase A, f ru ctose-bisp hos p hate (ALDOA), transcript variant 2, mRNA. | 0.1305 | -0.1957 |
| LOC729021 | PREDICTED: Homo sapiens hypothetical protein LOC729021 (LOC729021), mRNA. | 0.1297 | -0.1946 |
| LST1 | Homo sapiens leukocyte specific transcript 1 (LST1) ), transcript variant 1, mRNA. | 0.1283 | -0.1924 |
| ASXL2 | Homo sapiens additional sex combs like 2 (Drosophila) (ASXL2), mRNA. | -0.1278 | 0.1917 |
| EIF2AK2 | Homo sapiens eukaryotic translation initiation factor 2-alpha kinase 2 (EIF2AK2), mRNA. | -0.1274 | 0.1911 |
| MMP9 | Homo sapiens matrix metallopeptidase 9 (gelatinase B, 92kDa gelatinase, 92kDa type IV c | 0.126 | -0.1889 |
| IFIT2 | Homo sapiens interferon induced protein with tetratricopeptide repeats 2 (IFIT2), mRNA. | -0.125 | 0.1876 |
| CCDC72 | Homo sapiens coiled-coil domain containing 72 (CCDC72), mRNA | -0.1242 | 0.1864 |
| LOC642113 | PREDICTED: Homo sapiens similar to Ig kappa chain V-III region HAH precursor (LOC64 | 0.124 | -0.186 |
| IFI44L | Homo sapiens interferon-induced protein 44-like (IF144L), mRNA. | -0.1232 | 0.1848 |
| ISG15 | Homo sapiens ISG15 ubiquitin-like modifier (ISG15), mRNA. | -0.1231 | 0.1847 |
| LOC650276 | PREDICTED: Homo sapiens similar to 60S ribosomal protein L7 (LOC650276), mRNA. | -0.1226 | 0.1839 |
| LY96 | Homo sapiens lymphocyte antigen 96 (LY96), mRNA. | -0.1226 | 0.1838 |
| HINT1 | Homo sapiens histidine triad nucleotide binding protein 1 (HINT1), mRNA. | -0.122 | 0.183 |
| CLEC12A | Homo sapiens C-type lectin domain family 12, member A (CLEC12A), transcript variant 2, | -0.1219 | 0.1829 |
| BSG | Homo sapiens basigin (Ok blood group) (BSG), transcript variant 3, mRNA. | 0.1214 | -0.1821 |
| IF16 | Homo sapiens interferon, alpha-inducible protein 6 (IFI6), transcript variant 2, mRNA. | -0.1193 | 0.1789 |
| XAF1 | Homo sapiens XIAP associated factor 1 (XAF1), transcript variant 2, mRNA. | -0.1177 | 0.1766 |
| SNRPG | Homo sapiens small nuclear ribonucleoprotein polypeptide G (SNRPG), mRNA | -0.1.172 | 0.1758 |
| NCF1 | Homo sapiens neutrophil cytosolic factor 1, (chronic granulomatous disease, autosomal 1) | 0.117 | -0.1754 |
| PAD14 | Homo sapiens peptidyl arginine deiminase, type IV PADI4), mRNA. | 0.1162 | -0.1743 |
| LOC441763 | PREDICTED: Homo sapiens hypothetical LOC441763 (LOC441763), mRNA. | -0.1158 | 0.1736 |
| RPS15A | Homo sapiens ribosomal protein S15a (RPS15A), transcript variant 1, mRNA. | -0.1157 | 0.1736 |
| PSMA6 | Homo sapiens proteasome (prosome, macropain) subunit, alpha type, 6 (PSMA6), mRNA | -0.1145 | 0.1718 |
| VDAC1 | Homo sapiens voltage-dependent anion channel 1 (VDAC1), mRNA. | 0.1145 | -0.1718 |
| LOC653773 | PREDICTED: Homo sapiens similar to ribosomal protein L31 (LOC653773), mRNA. | -0.1131 | 0.1697 |
| ERAP2 | Homo sapiens endoplasmic reticulum aminopeptidase 2 (ERAP2), mRNA. | -0.113 | 0.1695 |
| SEC14L1 | Homo sapiens SEC14-like 1 (S. cerevisiae) (SEC14L1), mRNA. | 0.1128 | -0.1692 |
| COX7C | Homo sapiens cytochrome c oxidase subunit VIIc (COX7C), nuclear gene encoding mitocl | -0.1115 | 0.1673 |
| LOC284422 | PREDICTED: Homo sapiens similar to HSPC323 (LOC284422), mRNA. | 0.1114 | -0.167 |
| no symbol | full-length cDNA clone CSOCAP005YH21 of Thymus of Homo sapiens (human) | 0.1112 | -0.1668 |
| CD79A | Homo sapiens CD79a molecule, immunoglobulin-associated alpha (CD79A), transcript va | 0.1111 | -0.1667 |
| SDCBP | Homo sapiens syndecan binding protein (syntenin) (SDCBP), transcript variant 2, mRNA. | 0.1086 | -0.1628 |
| S100A4 | Homo sapiens S100 calcium binding protein A4(S100A4), transcript variant 2, mRNA. | 0.1057 | -0.1586 |
| ORM1 | Homo sapiens orosomucoid 1 (ORM1), mRNA | 0.1053 | -0.158 |
| LOC647450 | PREDICTED: Homo sapiens similar to Ig kappa chain V-I region HK101 precursor (LOC6∼ | 0.1051 | -0.1577 |
| LOC647673 | PREDICTED: Homo sapiens similar to Translationally-controlled tumor protein (TCTP) (p2 | -0.1049 | 0.1574 |
| UQCRQ | Homo sapiens ubiquinol-cytochrome c reductase, complex III subunitVII, 9.5kDa (UQCRC | -0.103 | 0.1544 |
| CLEC12A | Homo sapiens C-type lectin domain family 12, member A (CLEC12A), transcript variant 1, | -0.1005 | 0.1508 |

## Claims

1. An in vitro method for determining whether an individual has an increased risk of developing symptoms of Rheumatoid Arthritis comprising the steps of determining the expression level of at least the ISG15 gene in a sample obtained from said individual and comparing said expression level with a predetermined reference value wherein an increased level of expression of said ISG15 gene in said sample is indicative of an increased risk.

2. Method according to claim 1 wherein the expression level of at least one additional gene selected from the group consisting of the genes shown in table 8 is determined and compared to a predetermined reference value, wherein an increased or a decreased level of expression of said at least one other gene in said sample is indicative of an increased risk.

3. Method according to claim 3 wherein said at least one additional gene are at least 10 additional genes selected from the group consisting of the genes shown in table 8.

4. Method according to claims 2 or 3 wherein said additional gene or additional genes are selected from the group consisting of: EPSTI1 , IFI6, OAS3, IFI44L, RSAD2, IFIT11, MX1 , CD274, SERPING1 , IFI27, CD19, CD79A, CD79B, MS4A1 , FCRL5, DARC, BCL2L1, RBM38, BAG1, TESC, KLF1, ERAF, SELENBP1 , CCL5, IFNG, GZMH and NKG7.

5. A method according to any one of claims 1-4, further comprising testing a sample of said individual for a further factor associated with an increased risk for developing Rheumatoid Arthritis.

6. A method according to claim 5, wherein said further factor comprises detecting the presence of an anti-citrullinated protein/peptide antibody, and/or rheumatoid factor (RF).

7. A method according to claim 5 wherein said further factor comprises detecting an elevated level of MCP-1, IL-10, FGF2 and/or Flt-3L.

## Patentansprüche

1. In-vitro-Verfahren zur Bestimmung, ob bei einer Person ein erhöhtes Risiko vorliegt, Symptome einer rheumatoiden Arthritis zu entwickeln, umfassend die Schritte des Bestimmens des Expressionslevels von mindestens dem ISG15-Gen in einer von der Person entnommenen Probe, und Vergleichen der Expressionslevel mit einem vorbestimmten Referenzwert, wobei ein erhöhter Expressionslevel des ISG15-Gens in der Probe ein erhöhtes Risiko anzeigt.

2. Verfahren nach Anspruch 1, wobei der Expressionslevel von mindestens einem zusätzlichen Gen, ausgewählt aus der Gruppe von in Tabelle 8 gezeigten Genen, bestimmt wird und mit einem vorbestimmten Referenzwert verglichen wird, wobei ein erhöhter oder verminderter Expressionslevel von mindestens einem anderen Gen in der Probe ein erhöhtes Risiko anzeigt.

3. Verfahren nach Anspruch 3, wobei das mindestens eine zusätzlich Gen mindestens 10 zusätzliche Gene sind, ausgewählt aus der Gruppe, bestehend aus den in Tabelle 8 gezeigten Genen.

4. Verfahren nach den Ansprüchen 2 oder 3, wobei das zusätzliche Gen oder die zusätzlichen Gene ausgewählt sind aus der Gruppe bestehend aus: EPSTI1, IFI6, OAS3, IFI44L, RSAD2, IFIT1, MX1, CD274, SERPING1, IFI27, CD19, CD79A, CD79B, MS4A1, FCRL5, DARC, BCL2L1, RBM38, BAG1, TESC, KLF1, ERAF, SELENBP1, CCL5, IFNG, GZMH und NKG7.

5. Verfahren nach einem der Ansprüche 1-4, ferner umfassend das Untersuchen einer Probe der Person auf einen weiteren Faktor, verbunden mit einem erhöhten Risiko, rheumatoide Arthritis zu entwickeln.

6. Verfahren nach Anspruch 5, wobei der weitere Faktor das Nachweisen des Vorhandenseins eines anti-zitrulliniertes Protein/Peptid Antikörpers und/oder eines Rheumatoidfaktors (RF) umfasst.

7. Verfahren nach Anspruch 5, wobei der weitere Faktor das Nachweisen eines erhöhten Niveaus an MCP-1, IL-10, FGF2 und/oder Flt-3L umfasst.

## Revendications

1. Procédé *in vitro* pour déterminer si un individu présente un risque accru de développer des symptômes de polyarthrite rhumatoïde comprenant les étapes de détermination du taux d'expression au moins du gène ISG15 dans un échantillon obtenu à partir dudit individu et de comparaison dudit taux d'expression avec une valeur de référence prédéterminée où l'augmentation du taux d'expression dudit gène ISG15 dans ledit échantillon indique un risque accru.

2. Procédé selon la revendication 1, dans lequel le taux d'expression au moins d'un gène supplémentaire choisi dans le groupe constitué des gènes présentés dans le tableau 8 est déterminé et comparé à une valeur de référence prédéterminée, où une augmentation ou une diminution du taux d'expression dudit au moins un autre gène dans ledit échantillon indique un risque accru.

3. Procédé selon la revendication 3, dans lequel ledit au moins un gène supplémentaire consiste en au moins 10 gènes supplémentaires choisis dans le groupe constitué des gènes présentés dans le tableau 8.

4. Procédé selon les revendications 2 ou 3, dans lequel ledit gène supplémentaire ou lesdits gènes supplémentaires sont choisis dans le groupe constitué de : EPSTI1, IFI6, OAS3, IFI44L, RSAD2, IFIT1, MX1, CD274, SERPING1, IFI27, CD19, CD79A, CD79B, MS4A1, FCRL5, DARC, BCL2L1, RBM38, BAG1, TESC, KLF1, ERAF, SELENBP1, CCL5, IFNG, GZMH et NKG7.

5. Procédé selon l'une quelconque des revendications 1 à 4, comprenant en outre l'analyse d'un échantillon dudit individu pour un autre facteur associé à un risque accru de développer une polyarthrite rhumatoïde.

6. Procédé selon la revendication 5, dans lequel ledit autre facteur comprend la détection de la présence d'un anticorps anti-protéine/peptide citrulliné, et/ou du facteur rhumatoïde (FR).

7. Procédé selon la revendication 5, dans lequel ledit autre facteur comprend la détection d'un taux élevé de MCP-1, IL-10, FGF2 et/ou Flt-3L.
